(19) Europäisches Patentamt / European Patent Office / Office européen des brevets

(11) EP 2 662 365 A1

(12) EUROPEAN PATENT APPLICATION
published in accordance with Art. 153(4) EPC

(43) Date of publication:
**13.11.2013 Bulletin 2013/46**

(21) Application number: **12732174.3**

(22) Date of filing: **05.01.2012**

(51) Int Cl.:
*C07D 233/58* (2006.01) *C07D 403/04* (2006.01)
*C07D 403/14* (2006.01) *C07D 405/04* (2006.01)
*C09K 11/06* (2006.01) *H01L 51/50* (2006.01)

(86) International application number:
**PCT/JP2012/050087**

(87) International publication number:
**WO 2012/093688 (12.07.2012 Gazette 2012/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.01.2011 JP 2011002083**
**07.01.2011 JP 2011002084**

(71) Applicant: **Idemitsu Kosan Co., Ltd.**
**Chiyoda-ku**
**Tokyo 100-8321 (JP)**

(72) Inventor: **NUMATA Masaki**
**Sodegaura-shi**
**Chiba 299-0293 (JP)**

(74) Representative: **Gille Hrabal**
**Patentanwälte**
**Brucknerstrasse 20**
**40593 Düsseldorf (DE)**

# (54) IMIDAZOLE COMPOUND PRODUCTION METHOD, IMIDAZOLE COMPOUND, IMIDAZOLE-BASED COMPOUND, ORGANIC METAL COMPLEX, MATERIAL FOR ORGANIC ELECTROLUMINESCENT ELEMENT, ORGANIC ELECTROLUMINESCENT ELEMENT, DISPLAY DEVICE, AND LIGHTING DEVICE

(57) A manufacturing method of an imidazole compound represented by a formula (1) below includes reacting 1-arylimidazole with a halogen-atom substituted compound. For performing this reaction, in a reaction system, a mole number $N_{f(2)}$ [mol] of the halogen-atom substituted compound and a total volume $V_{sol}$ [liter] of an ether solvent having at most 5 carbon atoms satisfy a relationship of $V_{sol}/N_{f(2)} \leq 3$.

In the formula (1): $R_1$ and $R_4$ represent a substituent and the like; $Z_1$ represents a group of atoms necessary for forming a hydrocarbon cyclic group and the like; $R_2$ and $R_3$ represent a bond, a hydrogen atom or an aromatic hydrocarbon group; $Z_2$ represents a group of atoms necessary for forming a five-membered hydrocarbon ring and the like together with C-C; and m represents an integer of 1 to 5.

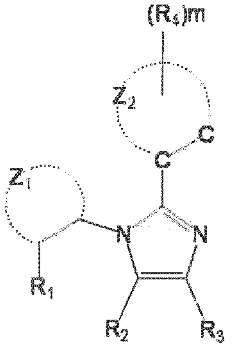

(1)



Printed by Jouve, 75001 PARIS (FR)

## Description

FIELD

Embodiments described herein relate generally to an imidazole compound manufacturing method, an imidazole compound, an imidazole-based compound, an organic metal complex, an organic-electroluminescence-device material, an organic electroluminescence device, a display device, and a lighting device.

BACKGROUND

An imidazole compound has been conventionally used for various usage, but, recently has been also used as an organic-electroluminescence-device material. For instance, an imidazole compound is used as a ligand of a metal complex.
Patent Literatures 1 and 2 disclose an N-phenyl-2-phenylimidazole derivative in which a substituent is introduced at each of second and sixth positions of a phenyl group of an N-position. Examples of the substituent are a methyl group, isopropyl group, phenyl group, 4-isopropyl phenyl group and 3,5-dimethylphenyl group. In Patent Literature 2, a steric bulkiness of the substituent is defined by a steric parameter (Es value).
Patent Literatures 1 and 2 disclose that, since a metal complex having as a ligand the imidazole compound in which a sterically bulky substituent is introduced at the second and sixth position of the phenyl group at the N-position has an emission wavelength shifted toward a short wavelength and exhibits a sharp emission spectrum, the metal complex is useful as a blue-emitting material having an excellent color purity.

CITATION LIST

PATENT LITERATURES

Patent Literature 1: JP-A-2008-542203
Patent Literature 2: JP-A-2008-303150

SUMMARY OF THE INVENTION

PROBLEMS TO BE SOLVED BY THE INVENTION

However, in synthesis methods disclosed in Patent Literatures 1 and 2, as the steric bulkiness of the substituent of the imidazole compound becomes larger (as the Es value becomes smaller), a yield rate is lowered and, further, the imidazole compound cannot be synthesized. For instance, when the substituent is a methyl group, an imidazole compound can be synthesized. However, an imidazole compound having an isopropyl group and a phenyl group exhibits an extremely low and unpractical yield rate. An imidazole compound having a carbazole group (i.e., a sterically more bulky substituent) cannot be synthesized. Although Patent Literatures 1 and 2 disclose synthesis examples of the imidazole compound having the isopropyl group and the phenyl group, but Patent Literatures 1 and 2 fail to disclose a synthesis example of an imidazole compound having a substituent having a steric bulkiness more than that of the isopropyl group and the phenyl group. Accordingly, Patent Literatures 1 and 2 cannot be recognized as providing an imidazole compound for practical use.
Accordingly, in order to obtain an emission material having a sharp emission spectrum and capable of emitting near a short wavelength, an imidazole compound having a substituent introduced at each of the second and sixth positions of the phenyl group at the N-position, and a method of manufacturing the imidazole compound at a higher yield rate are desired.
An object of the invention is to provide a method of manufacturing at a higher yield rate an imidazole compound useful for obtaining an emission material exhibiting a sharp emission spectrum and capable of emitting in or near a short wavelength; an imidazole-based compound; an organic metal complex; an organic-electroluminescence-device material containing the imidazole compound, the imidazole-based compound and/or the organic metal complex; an organic electroluminescence device using the organic-electroluminescence-device material; and a display device and a lighting device including the organic electroluminescence device.

MEANS FOR SOLVING THE PROBLEMS

According to an aspect of the invention, a manufacturing method of an imidazole compound represented by a formula (1) below includes: reacting a compound represented by a formula (2) below with a compound represented by a formula (3) below, in which when reacting the compound represented by the formula (2) with the compound represented by the formula (3), within a reaction system, a mole number $N_{f2}$ [mol] of the compound represented by the formula (2) and a total volume $V_A$ [liter] of an ether solvent having at most 5 carbon atoms satisfy a relationship of a numerical formula (1) below.

[Formula 1]

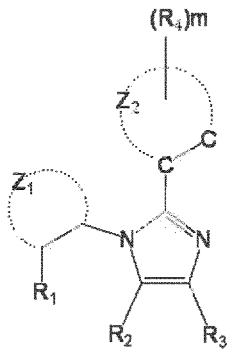

(1)

In the formula (1), $R_1$ represents a hydrogen atom or a substituent; and $Z_1$ represents a group of atoms necessary for forming a hydrocarbon cyclic group or a heterocyclic group, the hydrocarbon cyclic group or the heterocyclic group formed in $Z_1$ having at least one substituent represented by $R_1$; $R_2$ and $R_3$ represent a bond, a hydrogen atom or an aromatic hydrocarbon group. $R_2$ and $R_3$ may be mutually bonded to form a five-membered hydrocarbon ring, six-membered hydrocarbon ring, five-membered heterocyclic ring or six-membered heterocyclic ring, the rings optionally further having a substituent; $Z_2$ represents a group of atoms necessary for forming a five-membered hydrocarbon ring, six-membered hydrocarbon ring, five-membered heterocyclic ring or six-membered heterocyclic ring together with C-C; $R_4$ represents a hydrogen atom or a substituent; and m is an integer of 1 to 5.

[Formula 2]

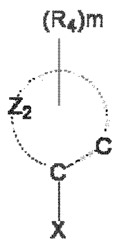

(2)

In the formula (2), X represents a halogen atom; and $Z_2$, $R_4$ and m represent the same as those in the formula (1).

[Formula 3]

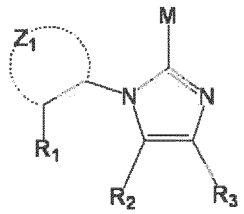

(3)

In the formula (3), M represents a boron atom, a magnesium atom, a silicon atom, a stannum atom and a zinc atom and optionally further has a substituent; $Z_1$, $R_1$, $R_2$ and $R_3$ represent the same as those in the formula (1).

[Numerical Formula 1]

$$V_A/N_{f2}\leq 3 \qquad (1)$$

In the manufacturing method of the imidazole compound according to the above aspect of the invention, the mole number $N_{f2}$ of the compound represented by the formula (2) and the total volume $V_A$ preferably satisfy a relationship of a numerical formula (2) below.

[Numerical Formula 2]

$$V_A/N_{f2}\leq 2 \qquad (2)$$

In the manufacturing method of the imidazole compound according to the above aspect of the invention, the mole number $N_{f2}$ of the compound represented by the formula (2) and the total volume $V_A$ preferably satisfy a relationship of a numerical formula (3) below.

[Numerical Formula 3]

$$V_A/N_{f2}\leq 1 \qquad (3)$$

In the manufacturing method of the imidazole compound according to the above aspect of the invention, the ether solvent having at most 5 carbon atoms is preferably at least one ether solvent selected from tetrahydrofuran, tetrahydropyran, 1,4-dioxane, 1,3-dioxane, diethylether and 1,2-dimethoxyethane.

The manufacturing method of the imidazole compound according to the above aspect of the invention preferably further includes: implementing a solvent removal treatment of removing a solvent in the reaction system to adjust the relationship between the mole number $N_{f2}$ and the total volume $V_A$.

In the manufacturing method of the imidazole compound according to the above aspect of the invention, preferably, as a second solvent, at least one solvent selected from an aliphatic hydrocarbon solvent having at least 7 carbon atoms, an aromatic hydrocarbon solvent and an ether solvent having at least 6 carbon atoms is contained in the reaction system, and when reacting the compound represented by the formula (2) with the compound represented by the formula (3), in the reaction system, the mole number $N_{f2}$ of the compound represented by the formula (2) and a total volume $V_B$ of the second solvent satisfy a relationship of a numerical formula (4) below.

[Numerical Formula 4]

$$0.1\leq V_B/N_{f2} \qquad (4)$$

In the manufacturing method of the imidazole compound according to the above aspect of the invention, in the reaction system, the mole number $N_{f2}$ of the compound represented by the formula (2) and the total volume $V_B$ of the second solvent preferably satisfy a relationship of a numerical formula (5) below.

[Numerical Formula 5]

$$0.1\leq V_B/N_{f2}\leq 10 \qquad (5)$$

In the manufacturing method of the imidazole compound according to the above aspect of the invention, the ether solvent having at least 6 carbon atoms is preferably at least one ether solvent selected from dipropylether, dibutylether, diethylene glycol dimethyl ether, diethylene glycol diethyl ether, methoxybenzene, ethoxybenzene, methyl anisole, ethyl anisole, dimethoxybenzene and methoxyethoxybenzene.
In the manufacturing method of the imidazole compound according to the above aspect of the invention, carbon atoms of the aliphatic hydrocarbon solvent having at least 7 carbon atoms are preferably in a range of 7 to 50, and carbon atoms of the aromatic hydrocarbon solvent are preferably in a range of 6 to 20.
In the manufacturing method of the imidazole compound according to the above aspect of the invention, the aromatic hydrocarbon solvent is preferably at least one solvent selected from benzene, toluene, xylene, ethylbenzene, trimethylbenzene and tetramethylbenzene.
In the manufacturing method of the imidazole compound according to the above aspect of the invention, in the formula (3), M is preferably a substituted or unsubstituted zinc atom.
An imidazole compound according to another aspect of the invention is manufactured by the manufacturing method of the imidazole compound according to the above aspect of the invention.
According to the above aspect of the invention, the imidazole compound includes a substituent represented by $R_1$ and having a steric parameter (Es) value of -2.0 or less in a formula (1) below.
[Formula 4]

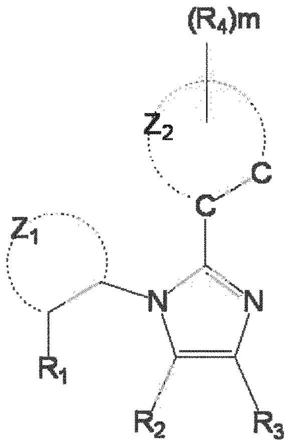

(1)

In the formula (1), $Z_1$ represents a group of atoms necessary for forming a hydrocarbon cyclic group or a heterocyclic group, the hydrocarbon cyclic group or the heterocyclic group formed in $Z_1$ having at least one substituent represented by $R_1$.
$R_2$ and $R_3$ represent a bond, a hydrogen atom or an aromatic hydrocarbon group and mutually bonded to form a five-membered hydrocarbon ring, six-membered hydrocarbon ring, five-membered heterocyclic ring or six-membered heterocyclic ring, the rings optionally further having a substituent.
$Z_2$ represents a group of atoms necessary for forming a five-membered hydrocarbon ring, six-membered hydrocarbon ring, five-membered heterocyclic ring or six-membered heterocyclic ring together with C-C.
$R_4$ represents a hydrogen atom or a substituent.
m is an integer of 1 to 5.
In the imidazole compound according to the above aspect of the invention, $R_1$ preferably represents a substituent having a steric parameter (Es) value of -2.5 or less.
In the imidazole compound according to the above aspect of the invention, $R_1$ preferably represents a substituent having a steric parameter (Es) value of -3.0 or less.
In the imidazole compound according to the above aspect of the invention, $R_1$ preferably represents a substituent having a steric parameter (Es) value of -5.0 or less.
In the imidazole compound according to the above aspect of the invention, $R_1$ preferably represents a substituent having a molecular weight of 42 or more.
In the imidazole compound according to the above aspect of the invention, $R_1$ preferably represents a substituent having a molecular weight of 76 or more.
In the imidazole compound according to the above aspect of the invention, $R_1$ preferably represents a substituent having a molecular weight of 115 or more.
In the imidazole compound according to the above aspect of the invention, $R_1$ preferably represents a substituent

having a molecular weight of 166 or more.

An imidazole-based compound according to still another aspect of the invention includes the imidazole compound according to the above aspect of the invention as a partial structure.

An organic metal complex according to a further aspect of the invention includes: the imidazole compound according to the above aspect of the invention as a partial structure.

The organic metal complex according to the above aspect of the invention preferably includes at least one metal selected from metal elements from the eighth group to the eleventh group in the periodic table.

An organic-electroluminescence-device material according to a still further aspect of the invention includes: at least one of the imidazole compound according to the aspect of the invention, the imidazole-based compound according to the aspect of the invention and the organic metal complex according to the aspect of the invention.

An organic electroluminescence device according to a still further aspect of the invention includes: a cathode; an anode; and a plurality of organic compound layers provided between the cathode and the anode, the organic compound layers comprising an emitting layer, in which at least one layer of the organic compound layers includes the organic-electroluminescence-device material according to the above aspect of the invention.

A display device according to a still further aspect of the invention includes: the organic electroluminescence device according to the above aspect of the invention.

A lighting device according to a still further aspect of the invention includes: the organic electroluminescence device according to the above aspect of the invention.

According to the invention, a method of manufacturing at a higher yield rate an imidazole compound useful for obtaining an emission material exhibiting a sharp emission spectrum and capable of emitting in or near a short wavelength; the imidazole compound; an imidazole-based compound having the imidazole compound as a partial structure; an organic metal complex; an organic-electroluminescence-device material containing the imidazole compound, the imidazole-based compound and/or the organic metal complex; an organic electroluminescence device using the organic-electroluminescence-device material; and a display device and a lighting device including the organic electroluminescence device can be provided.

BRIEF DESCRIPTION OF DRAWING

Fig. 1 shows a $^1$H-NMR spectrum of an imidazole compound.
Fig. 2 shows a partially enlarged view of the $^1$H-NMR spectrum in Fig. 1
Fig. 3A shows an analysis result by a high speed liquid chromatography.
Fig. 3B shows an analysis result by a high speed liquid chromatography.

DESCRIPTION OF EMBODIMENTS

First Exemplary Embodiment

A first exemplary embodiment of the invention will be described in detail below.

Imidazole Compound

An imidazole compound according to the exemplary embodiment is represented by the formula (1).
In the formula (1), $Z_1$ represents a group of atoms necessary for forming a hydrocarbon cyclic group or a heterocyclic group. The hydrocarbon cyclic group or the heterocyclic group has at least one substituent represented by $R_1$ described below. The number of $R_1$ is preferably 1, 2, 3, 4 or 5. When the number of $R_1$ is plural, $R_1$ may be mutually the same or different. Among the imidazole compound represented by the formula (1), an imidazole compound having $R_1$ at each of ortho positions of the hydrocarbon cyclic group or the heterocyclic group is preferable.

$R_1$ represents a hydrogen atom or a substituent.
Examples of the substituent represented by $R_1$ include: an alkyl group (e.g., a methyl group, ethyl group, propyl group, isopropyl group, tert-butyl group, pentyl group, hexyl group, octyl group, dodecyl group, tridecyl group, tetradecyl group and pentadecyl group); a cycloalkyl group (e.g., a cyclopentyl group and a cyclohexyl group); an alkenyl group (e.g., a vinyl group and an allyl group); an alkynyl group (e.g., ethynyl group and a propargyl group); an aromatic hydrocarbon group (also referred to as an aromatic hydrocarbon cyclic group, an aromatic carbon cyclic group, an aryl group or the like) (e.g., a phenyl group, p-chlorophenyl group, mesityl group, tolyl group, xylyl group, naphthyl group, anthryl group, azulenyl group, acenaphthenyl group, fluorenyl group, phenanthryl group, indenyl group, pyrenyl group and biphenylyl group); an aromatic heterocyclic group (e.g., a pyridyl group, pyrimidinyl group, furyl group, pyrrolyl group, imidazolyl

group, benzimidazolyl group, pyrazolyl group, pyrazinyl group, triazolyl group (e.g., 1,2,4-triazole-1-yl group and 1,2,3-triazole-1-yl group), oxazolyl group, benzoxazolyl group, thiazolyl group, isoxazolyl group, isothiazolyl group, furazanyl group, thienyl group, quinolyl group, benzofuryl group, dibenzofuryl group, benzothienyl group, dibenzothienyl group, indolyl group, carbazolyl group, carbolinyl group, diazacarbazolyl group (a group obtained by substituting one of carbon atoms forming a carboline ring of the carbolinyl group with a nitrogen atom), quinoxalinyl group, pyridazinyl group, triazinyl group, quinazolinyl group and phthalazinyl group); a heterocyclic group (e.g., a pyrrolidyl group, imidazolydyl group, morpholyl group and oxazolydyl group); an alkoxy group (e.g., methoxy group, ethoxy group, propyloxy group, pentyloxy group, hexyloxy group, octyloxy group and dodecyloxy group); a cycloalkoxy group (e.g., a cyclopentyloxy group and a cyclohexyloxy group); an aryloxy group (e.g., a phenoxy group and a naphthyloxy group); an alkylthio group (e.g., a methylthio group, ethylthio group, propylthio group, pentylthio group, hexylthio group, octylthio group and dodecylthio group); a cycloalkylthio group (e.g., a cyclopentylthio group and a cyclohexylthio group); an arylthio group (e.g., a phenylthio group and a naphthylthio group); an alkoxycarbonyl group (e.g., a methyloxycarbonyl group, ethyloxycarbonyl group, butyloxycarbonyl group, octyloxycarbonyl group and dodecyloxycarbonyl group); an aryloxycarbonyl group (e.g., a phenyloxycarbonyl group and a naphthyloxycarbonyl group); a sulfamoyl group (e.g., an aminosulfonyl group, methylaminosulfonyl group, dimethylaminosulfonyl group, butylaminosulfonyl group, hexylaminosulfonyl group, cyclohexylaminosulfonyl group, octylaminosulfonyl group, dodecylaminosulfonyl group, phenylaminosulfonyl group, naphthylaminosulfonyl group and 2-pyridylaminosulfonyl group); an acyl group (e.g., an acetyl group, ethylcarbonyl group, propylcarbonyl group, pentylcarbonyl group, cyclohexylcarbonyl group, octylcarbonyl group, 2-ethylhexylcarbonyl group, dodecylcarbonyl group, phenylcarbonyl group, naphthylcarbonyl group and pyridylcarbonyl group); an acyloxy group (e.g., an acetyloxy group, ethylcarbonyloxy group, butylcarbonyloxy group, octylcarbonyloxy group, dodecylcarbonyloxy group and phenylcarbonyloxy group); an amido group (e.g., a methylcarbonylamino group, ethylcarbonylamino group, dimethylcarbonylamino group, propylcarbonylamino group, pentylcarbonylamino group, cyclohexylcarbonylamino group, 2-ethylhexylcarbonylamino group, octylcarbonylamino group, dodecylcarbonylamino group, phenylcarbonylamino group and naphtylcarbonylamino group); a carbamoyl group (e.g., an aminocarbonyl group, methylaminocarbonyl group, dimethylaminocarbonyl group, propylaminocarbonyl group, pentylaminocarbonyl group, cyclohexylaminocarbonyl group, octylaminocarbonyl group, 2-etylhexylaminocarbonyl group, dodecylaminocarbonyl group, phenylaminocarbonyl group, naphthylaminocarbonyl group and 2-pyridylaminocarbonyl group); a ureido group (e.g., a methylureido group, ethylureido group, pentylureido group, cyclohexylureido group, octylureido group, dodecylureido group, phenylureido group, naphthylureido group and 2-pyridylaminoureido group); a sulfinyl group (e.g., a methylsulfinyl group, ethylsulfinyl group, butylsulfinyl group, cyclohexylsulfinyl group, 2-ethylhexylsulfinyl group, dodecylsulfinyl group, phenylsulfinyl group, naphthylsulfinyl group and 2-pyridylsulfinyl group); an alkylsulfonyl group (e.g., a methylsulfonyl group, ethylsulfonyl group, butylsulfonyl group, cyclohexylsulfonyl group, 2-ethylhexylsulfonyl group and dodecylsulfonyl group); an arylsulfonyl group or a heteroarylsulfonyl group (e.g., a phenylsulfonyl group, naphthylsulfonyl group and 2-pyridylsulfonyl group); an amino group (e.g., an amino group, ethylamino group, dimethylamino group, butylamino group, cyclopentylamino group, 2-ethylhexylamino group, dodecylamino group, anilino group, naphthylamino group and 2-pyridylamino group); a halogen atom (e.g., a fluorine atom, chlorine atom and bromine atom); a fluorohydrocarbon group (e.g., a fluoromethyl group, trifluoromethyl group, pentafluoroethyl group and pentafluorophenyl group); a cyano group; a nitro group; a hydroxy group; a mercapto group; a silyl group (e.g., a trimethylsilyl group, triisopropylsilyl group, triphenylsilyl group and phenyldiethylsilyl group); and a phosphono group.

In the formula (1), $R_1$ is preferably a substituent having a steric parameter (Es) value of -1.70 or less, more preferably of -2.0 or less, further preferably of -2.5 or less, still further preferably of -3.0 or less, particularly preferably of -5.0 or less.

The Es value is a steric parameter induced from chemical reactivity. It can be said that the smaller the Es value is, the more sterically bulky the substituent is. Accordingly, the smaller Es value is preferable.

The Es value is described in, for instance, "Structure-activity relationships of drug: The significance in drug design and mode-of-action studies (special number 122 of Kagaku no Ryoiki) (page 124-126)" edited by Kozo Kassei Konwakai and published by Nankodo Co., Ltd. in 1979) and "American Chemical Society Professional Reference Book "Exploring QSAR" p.81 Table 3-3" in Unger, S.H., Hansch, C., Prog. Phys. Org. Chem., 12,91 (1976)."

The Es value in the exemplary embodiment is a value that is represented as zero for a hydrogen atom.

The substituent having the Es value of -2.0 or less is exemplified by $-CF_3$ (a trifluoromethyl group), a 9-H-fluorene group (an Es value =-2.34) and a carbazolyl group. The substituent having the Es value of -2.5 or less is exemplified by $-t-C_4H_9$ (a tert-butyl group). The substituent having the Es value of -3.0 or less is exemplified by $-CH(C_2H_5)_2$ (a 3-(n-pentyl) group), $-CHBr_2$ (a dibromomethyl group), $-CCl_3$ (a trichloromethyl group) and $-CBr_3$ (a tribromomethyl group). The substituent having the Es value of -5.0 or less is exemplified by $-C(C_6H_5)_3$ (a triphenylmethyl group). These substituents may be further substituted by the substitutent $R_1$.

In the formula (1), in addition to falling within the range of the Es value, $R_1$ is preferably a substituent having a molecular weight of 43 or more, more preferably of 77 or more, further preferably of 116 or more, still further preferably of 127 or more, still further preferably of 166 or more. The substituent having a molecular weight of 43 or more is

exemplified by an isopropyl group (molecular weight: 43). The substituent having a molecular weight of 77 or more is exemplified by a phenyl group (molecular weight: 77). The substituent having a molecular weight of 116 or more is exemplified by an indole group (molecular weight: 116). The substituent having a molecular weight of 127 or more is exemplified by a naphthyl group (molecular weight: 127). The substituent having a molecular weight of 166 or more is exemplified by a carbazole group (molecular weight: 166).

Hydrocarbon Cyclic Group

As described above, in the formula (1), $Z_1$ represents a group of atoms necessary for forming a hydrocarbon cyclic group. The hydrocarbon cyclic group is exemplified by a cycloalkyl group or an aryl group (aromatic cyclic group).

Cycloalkyl Group

Examples of the cycloalkyl group are a cyclopropyl group, cyclobutyl group, cyclopentyl group, cyclohexyl group, cycloheptyl group, cyclooctyl group, cyclododecyl group and norbornyl group. The cycloalkyl group preferably has carbon atoms of 5 to 10, more preferably of 5 to 7.

Aryl Group

Examples of the aryl group are a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, biphenyl-2-yl group, biphenyl-3-yl group, biphenyl-4-yl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenyl-4-yl group, 4"-t-butyl-p-terphenyl-4-yl group and fluorenyl group. The aryl group preferably has carbon atoms of 6 to 18, more preferably of 6 to 12.

Heterocyclic Group

As described above, in the formula (1), $Z_1$ represents a group of atoms necessary for forming a heterocyclic group. The heterocyclic group is exemplified by an aliphatic heterocyclic group and an aromatic heterocyclic group.

Aliphatic Heterocyclic Group

Examples of the aliphatic heterocyclic group are ones derived from an epoxy ring, aziridine ring, thiirane ring, oxetane ring, azetidine ring, thietane ring, tetrahydrofuran ring, dioxolane ring, pyrrolidine ring, pyrazolidine ring, imidazolidine ring, oxazolidine ring, tetrahydrothiophene ring, sulfolane ring, thiazolidine ring, ε-caprolactone ring, ε-caprolactam ring, piperidine ring, hexahydropyridazine ring, hexahydropyrimidine ring, piperazine ring, morpholine ring, tetrahydropyran ring, 1,3-dioxane ring, 1,4-dioxane ring, trioxane ring, tetrahydrothiopyran ring, thiomorpholine ring, thiomorpholine-1, 1-dioxide ring, pyranose ring and diazabicyclo[2,2,2]-octane ring. The aliphatic heterocyclic group preferably has ring atoms of 5 to 10, more preferably of 5 to 7.

Aromatic Heterocyclic Group

Examples of the aromatic heterocyclic group are a pyridyl group, pyrimidinyl group, furyl group, pyrrolyl group, imidazolyl group, benzimidazolyl group, pyrazolyl group, pyrazinyl group, triazolyl group (e.g., 1,2,4-triazole-1-yl group and 1,2,3-triazole-1-yl group), oxazolyl group, benzoxazolyl group, thiazolyl group, isooxazolyl group, isothiazolyl group, furazanyl group, thienyl group, quinolyl group, benzofuryl group, dibenzofuryl group, benzothienyl group, dibenzothienyl group, indolyl group, carbazolyl group, carbolinyl group, diazacarbazolyl group (a group obtained by substituting one of carbon atoms forming a carboline ring of the carbolinyl group with a nitrogen atom), quinoxalinyl group, pyridazinyl group, triazinyl group, quinazolinyl group and phthalazinyl group. The aromatic heterocyclic group preferably has ring atoms of 5 to 18, more preferably of 5 to 13.

In the formula (1), $R_2$ and $R_3$ represent a bond, a hydrogen atom or an aromatic hydrocarbon group. $R_2$ and $R_3$ may be mutually bonded to form a five-membered hydrocarbon ring, six-membered hydrocarbon ring, five-membered heterocyclic ring or six-membered heterocyclic ring. Further, these rings may have the substituent $R_1$.
Examples of the five- or six-membered hydrocarbon ring are a cyclopentane ring, cyclopentadiene ring, cyclohexane ring, cyclohexadiene ring and benzene ring.

Examples of the five- or six-membered heterocyclic ring include: a five- or six-membered aromatic heterocyclic ring (e.g., an oxazole ring, oxadiazole ring, oxatriazole ring, isooxazole ring, tetrazole ring, thiadiazole ring, thiatriazole ring, isothiazole ring, thiophene ring, furan ring, pyrrole ring, pyridine ring, pyridazine ring, pyrimidine ring, pyrazine ring, triazine ring, imidazole ring and pyrazole ring and triazole ring); and a five-or six-membered non-aromatic heterocyclic ring (e.g., a pyrrolidine ring, piperazine ring, pyrazolidine ring, imidazolidine ring, isooxazolidine ring and isothiazolidine ring).

In the formula (1), $Z_2$ represents a group of atoms necessary for forming a five-membered hydrocarbon ring, six-membered hydrocarbon ring, five-membered heterocyclic ring or six-membered heterocyclic ring together with C-C. The five-membered hydrocarbon ring, six-membered hydrocarbon ring, five-membered heterocyclic ring and six-membered heterocyclic ring are the same as those described in relation to $R_2$ and $R_3$.

In the formula (1), $R_4$ represents a hydrogen atom or a substituent. The substituent is exemplified by the substituent $R_1$.

In the formula (1), m is an integer of 1 to 5. When the number of $R_4$ is 2 or more, $R_4$ may be mutually the same or different.

In the formula (1), the hydrocarbon cyclic group formed by $Z_1$ is preferably an aryl group, more preferably a phenyl group.

Further, second and sixth positions of the phenyl group are preferably substituted by the substituent $R_1$.

Examples of specific structures of the imidazole compound according to the exemplary embodiment are as follows. However, the invention is not limited to the imidazole compound having these structures.

[Formula 5]

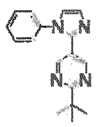
L-1

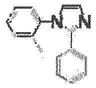
L-2

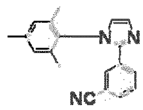
L-3

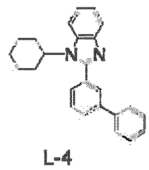
L-4

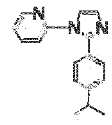
L-5

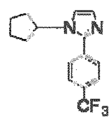
L-6

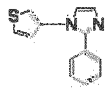
L-7

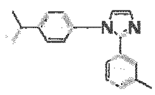
L-8

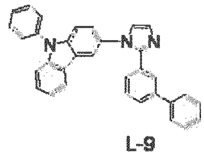
L-9

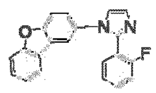
L-10

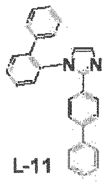
L-11

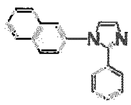
L-12

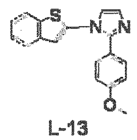
L-13

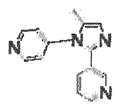
L-14

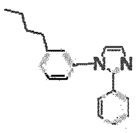
L-15

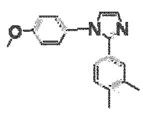
L-16

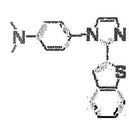
L-17

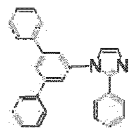
L-18

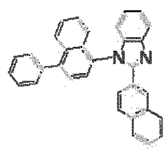
L-19

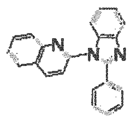
L-20

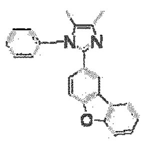
L-21

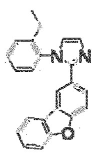
L-22

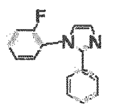
L-23

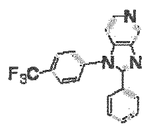
L-24

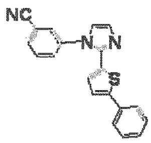
L-25

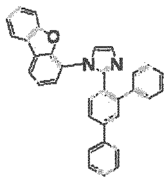
L-26

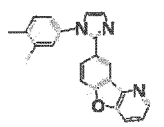
L-27

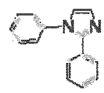
L-28

[Formula 6]

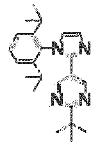
L-29

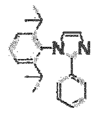
L-30

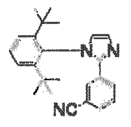
L-31

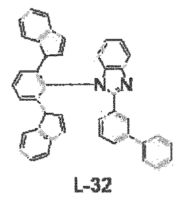
L-32

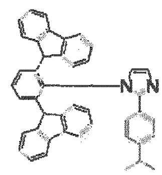
L-33

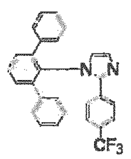
L-34

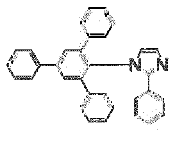
L-35

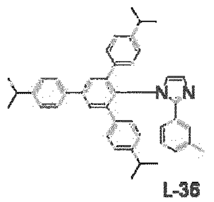
L-36

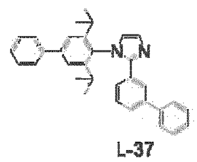
L-37

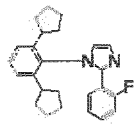
L-38

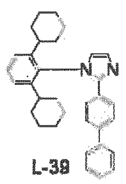
L-39

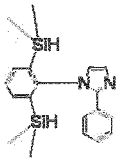
L-40

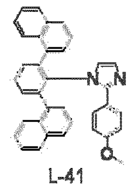
L-41

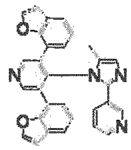
L-42

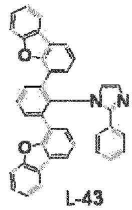
L-43

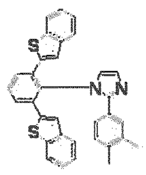
L-44

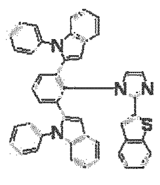
L-45

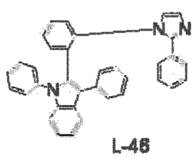
L-46

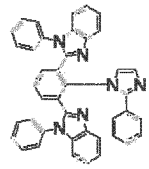
L-47

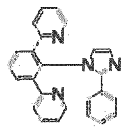
L-48

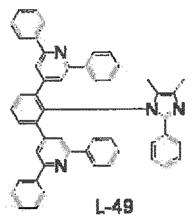
L-49

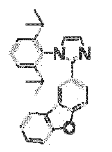
L-50

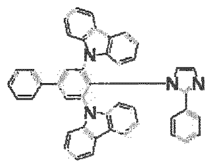
L-51

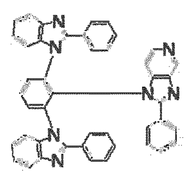
L-52

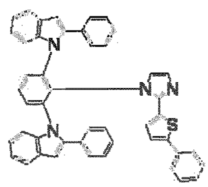
L-53

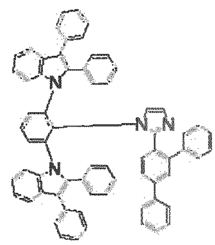
L-54

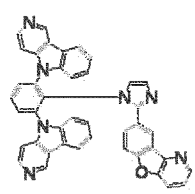
L-55

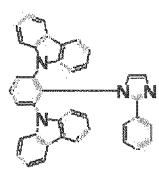
L-56

[Formula 7]

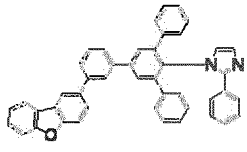
L-57

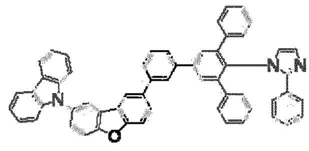
L-58

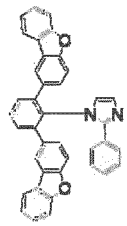
L-59

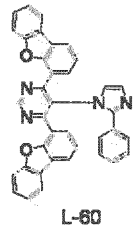
L-60

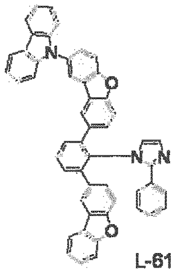
L-61

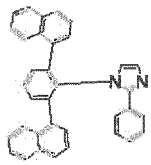
L-62

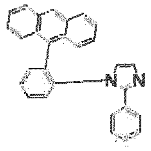
L-63

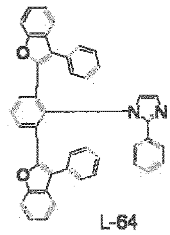
L-64

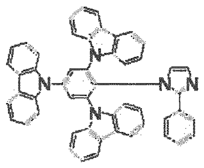
L-65

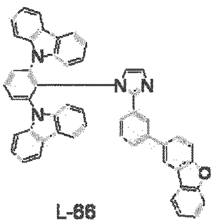
L-66

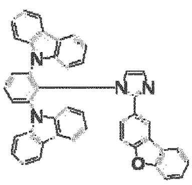
L-67

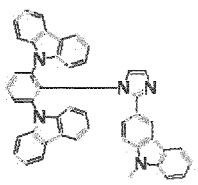
L-68

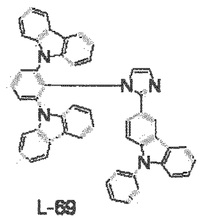
L-69

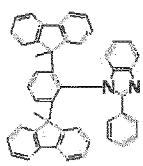
L-70

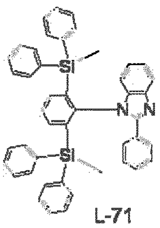
L-71

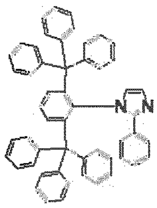
L-72

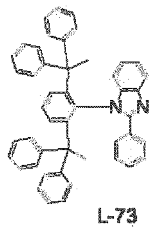
L-73

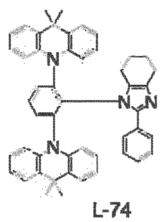
L-74

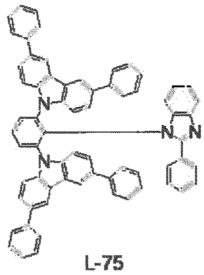
L-75

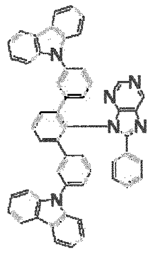
L-76

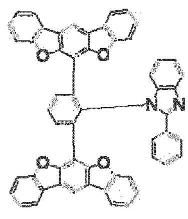
L-77

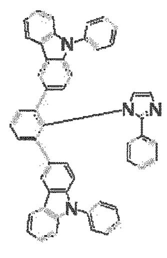
L-78

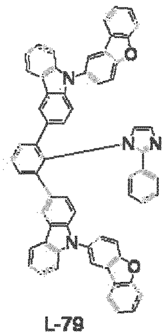
L-79

[Formula 8]

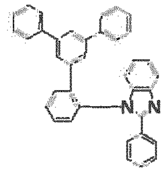
L-80

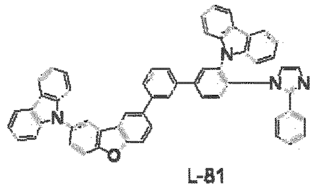
L-81

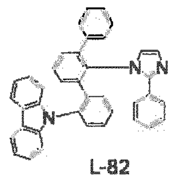
L-82

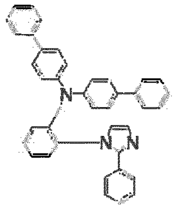
L-83

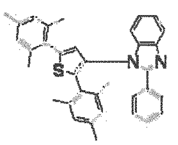
L-84

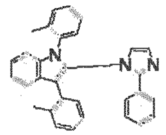
L-85

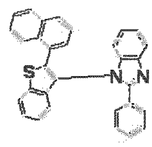
L-86

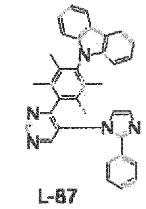
L-87

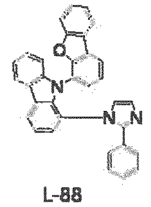
L-88

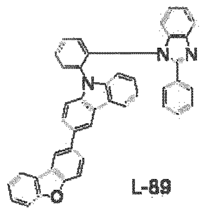
L-89

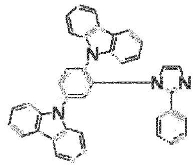
L-90

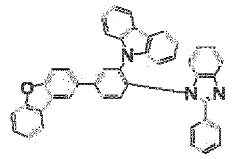
L-91

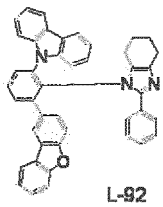
L-92

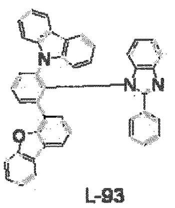
L-93

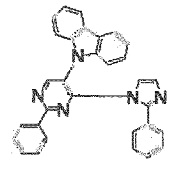
L-94

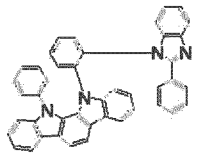
L-95

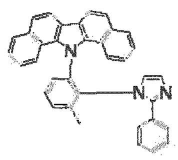
L-96

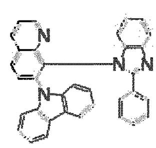
L-97

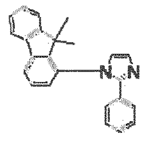
L-98

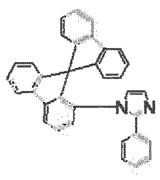
L-99

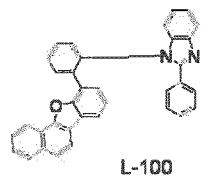
L-100

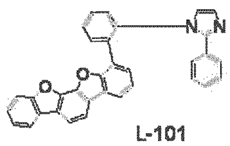
L-101

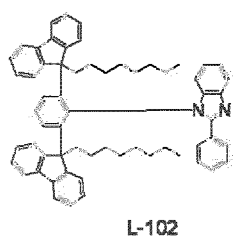
L-102

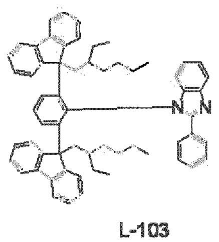
L-103

[Formula 9]

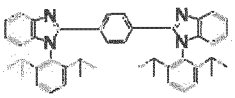
L-104

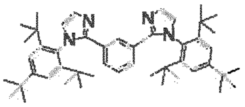
L-105

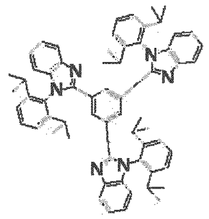
L-106

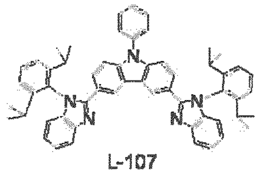
L-107

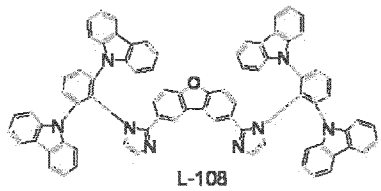
L-108

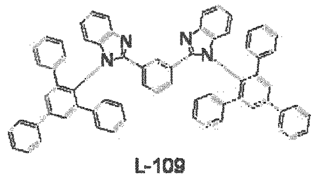
L-109

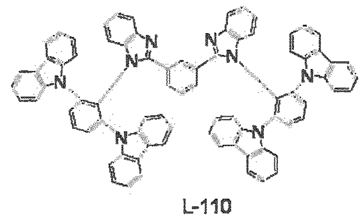
L-110

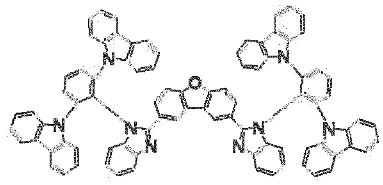
L-111

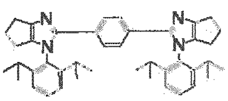
L-112

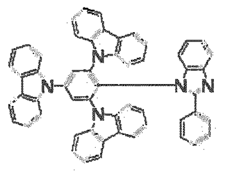
L-113

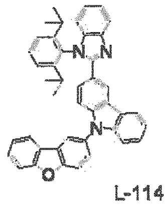
L-114

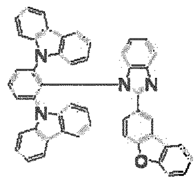
L-115

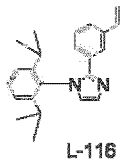
L-116

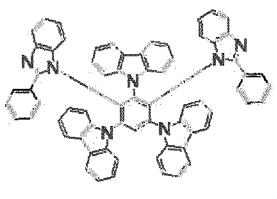
L-117

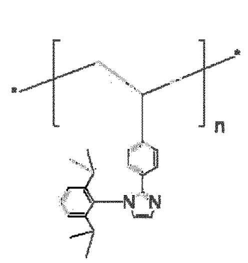
L-118

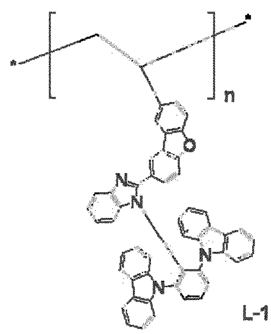
L-119

Manufacturing Method of Imidazole Compound

A manufacturing method of the imidazole compound according to the exemplary embodiment is performed by reacting the compound represented by the formula (2) and the compound represented by the formula (3) (hereinafter, referred to as Reaction 1), thereby manufacturing the imidazole compound satisfying the numerical formula (1) and represented by the formula (1). For performing the Reaction 1, in a reaction system, a mole number $N_{f(2)}$ [mol] of the compound represented by the formula (2) and a total volume $V_A$ [liter] of an ether solvent having at most 5 carbon atoms satisfy the relationship of the numerical formula (1).

In the formula (3), M is preferably a zinc atom that may have a substituent. In such a case, a synthesis reaction of the compound represented by the formula (3) (i.e., a synthesis reaction prior to the Reaction 1) and the Reaction 1,

namely, a two-step synthesis reaction in terms of reaction mechanism can be continuously performed (also referred to as "1 pot synthesis"), which is preferable since manufacturing steps can be simplified.

When plural kinds of the ether solvents having at most 5 carbon atoms are contained in the reaction system, $V_A$ represents a total volume of the plural kinds of the ether solvents.

The manufacturing method of the imidazole compound according to the exemplary embodiment is preferably performed under the condition of satisfying the relationship of the numerical formula (2), more preferably under the condition of satisfying the relationship of the numerical formula (3).

Examples of the ether solvent having at most 5 carbon atoms are tetrahydrofuran, tetrahydropyran, 1,4-dioxane, 1,3-dioxane, diethylether and 1,2-dimethoxyethane. This solvent can also be defined as an ether solvent having 2 to 5 carbon atoms.

Any one of the numerical formulae (1) to (3) is satisfied by adjusting the volume of the ether solvent having at most 5 carbon atoms which is added in preparation for the Reaction 1. When the following numerical formula (6) is satisfied at the time of performing the Reaction 1 due to solubility of a reagent used for the Reaction 1 and steps prior to the Reaction 1, a solvent removal treatment is conducted against the reaction system, thereby adjusting the volume of the ether solvent having at most 5 carbon atoms so as to satisfy any one of the numerical formulae (1) to (3).

[Numerical Formula 6]

$$V_A/N_{f2}>3 \cdots(6)$$

Usable methods for the solvent removal treatment include: a vacuum distillation method in which a solvent is removed under reduced pressure using a solvent-trapping vessel that is cooled by various cooling systems and using various vacuum pumps; and an atmospheric distillation method in which a solvent is removed under atmospheric pressure using Dean-Stark trap.

In the manufacturing method of the imidazole compound according to the exemplary embodiment, in addition to satisfying any one of the formulae (1) to (3), it is preferable that at least one solvent selected from an ether solvent having at least 6 carbon atoms, an aliphatic hydrocarbon solvent having at least 7 carbon atoms and an aromatic hydrocarbon solvent is contained in the reaction system.

In this case, for performing the Reaction 1, a total volume $V_B$ [liter] of the at least one solvent selected from the ether solvent having at least 6 carbon atoms, the aliphatic hydrocarbon solvent having at least 7 carbon atoms and the aromatic hydrocarbon solvent and a mole number $N_{f2}$ [mol] of the compound represented by the formula (2) preferably satisfy the relationship of the numerical formula (4), more preferably of the numerical formula (5).

Preferable examples of the ether solvent having at least 6 carbon atoms are dipropylether, dibutylether, diethylene glycol dimethyl ether, diethylene glycol diethyl ether, methoxybenzene, ethoxybenzene, methyl anisole, ethyl anisole, dimethoxybenzene and methoxyethoxybenzene. The ether solvent having at least 6 carbon atoms is preferably an ether solvent having 6 to 30 carbon atoms, more preferably 8 to 15 carbon atoms.

The aliphatic hydrocarbon solvent having at least 7 carbon atoms is preferably selected from an aliphatic hydrocarbon solvent having 7 to 50 carbon atoms, preferable examples of which are n-heptane, n-octane, n-nonane and n-decane. More preferably, the aliphatic hydrocarbon solvent has 8 to 30 carbon atoms.

The aromatic hydrocarbon solvent is preferably selected from an aromatic hydrocarbon solvent having 6 to 20 carbon atoms, preferable examples of which are benzene, toluene, xylene, ethylbenzene, trimethylbenzene and tetramethylbenzene. More preferably, the aromatic hydrocarbon solvent has 7 to 10 carbon atoms.

In the manufacturing method of the imidazole compound according to the exemplary embodiment, the Reaction 1 is preferably performed in an organic solvent, as needed, in the presence of at least one of an inorganic basic compound and an organic basic compound as well as in the presence of a metal catalyst.

Examples of the inorganic basic compound used as needed for the Reaction 1 are sodium carbonate, potassium carbonate, cesium carbonate and potassium phosphate.

Examples of the organic basic compound used as needed for the Reaction 1 are sodium acetate, potassium acetate, sodium tert-butoxide and potassium tert-butoxide.

Preferable examples of the metal catalyst used for the Reaction 1 are a palladium catalyst and a nickel catalyst. A palladium compound having a phosphine ligand is preferably used as the palladium catalyst, examples of which are $Pd(PPh_3)_4$ and $PdCl_2(PPh_3)_2$.

By mixing a palladium compound containing no phosphine ligand such as $Pd(OAc)_2$, $Pd(dba)_2$ and $Pd_2(dba)_3$ with a phosphine ligand such as $PPh_3$, tricyclohexylphosphine, tri-tert-butylphosphine, dppe, dppp and dppf in the reaction system, a palladium compound having a phosphine ligand can be prepared in the reaction system.

In addition to the above palladium catalyst, a palladium catalyst used for forming carbon-to-carbon (C-C) bond which is known to those skilled in the art is preferably used.

A nickel compound having a phosphine ligand is preferably used as the nickel catalyst, examples of which are $NiCl_2$ (dppe), $NiCl_2$(dppf) and $NiCl_2(PPh_3)_2$. In addition to the above nickel catalyst, a nickel catalyst used for forming C-C bond which is known to those skilled in the art is preferably used.

According to the above-described manufacturing method of the imidazole compound according to the exemplary embodiment, such an imidazole compound as having a low yield rate or being incapable of being synthesized according to a conventionally disclosed synthesis method can be manufactured at a high yield rate.

The reasons are speculated as follows.

An ether solvent consists of molecules formed of an oxygen atom and a hydrocarbon group. In accordance with increase in the number of carbon atoms of the hydrocarbon group, hydrophobicity of the solvent is increased. For instance, this point is described in "Surfactant-Property, Application, Chemical Ecology" (published by Kodansha Ltd. (1979, First issue)) and "New Surfactant" (published by SANKYO PUBLISHING Co., Ltd. (1986, Fourth issue)).

As shown in later-described Examples, when the Reaction 1 is performed with a great volume of tetrahydrofuran having 4 carbon atoms (i.e., a reaction solvent) in the reaction system (i.e., under the condition that the relationship of the numerical formula (1) is not satisfied), the imidazole compound represented by the formula (1) is synthesized at a low yield rate or impossible to be synthesized. On the other hand, by decreasing the volume of tetrahydrofuran existing in the reaction system or decreasing a ratio of tetrahydrofuran in the reaction solvent by adding toluene in the reaction system, in other words, by performing a reaction under the condition that the relationship of the numerical formula (1) is satisfied or by performing a reaction under the condition that the relationship of the numerical formula (1) is satisfied and the relationship of the numerical formula (4) is satisfied, the yield rate of the imidazole compound is drastically improved. For this reason, it is speculated that hydrophobicity of the solvent existing in the reaction system significantly affects the yield rate of the imidazole compound.

In other words, when a large volume of the ether solvent having at most 5 carbon atoms exists in the reaction system, the yield rate of the imidazole compound is low. However, by decreasing the volume of the ether solvent having at most 5 carbon atoms existing in the reaction system or decreasing the ratio of tetrahydrofuran in the reaction solvent by adding to the reaction system at least one solvent selected from the ether solvent having at least 6 carbon atoms, the aliphatic hydrocarbon solvent having at least 7 carbon atoms and the aromatic hydrocarbon solvent, the imidazole compound represented by the formula (1) can be synthesized at a high yield rate.

Imidazole-based Compound

Using the imidazole compound manufactured according to the manufacturing method of the imidazole compound according to the exemplary embodiment, an imidazole-based compound having the imidazole compound as a partial structure can be obtained.

The imidazole-based compound according to the exemplary embodiment has the imidazole compound represented by the formula (1) as a partial structure. In other words, as long as a compound, even partially, has the structure represented by the formula (1), the compound falls under the imidazole-based compound according to the exemplary embodiment. Accordingly, the multi-substituted compounds such as L-104 to L-119, which are examples of the imidazole compound, also fall under the imidazole-based compound according to the exemplary embodiment.

Specific examples of the imidazole-based compound according to the exemplary embodiment will be additionally shown below.

[Formula 10]

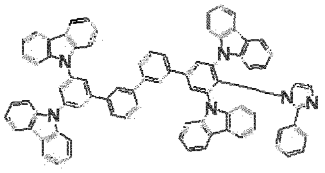

L-120

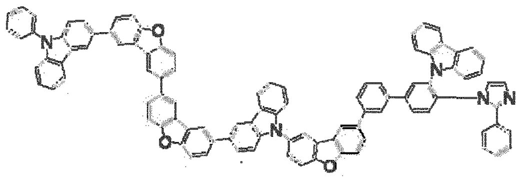

L-121

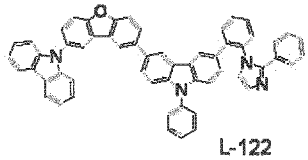

L-122

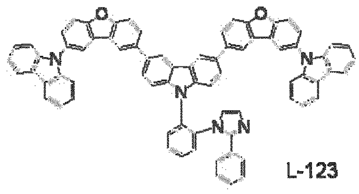

L-123

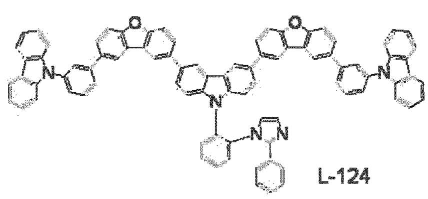

L-124

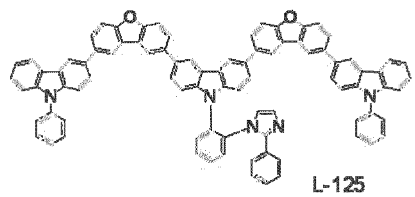

L-125

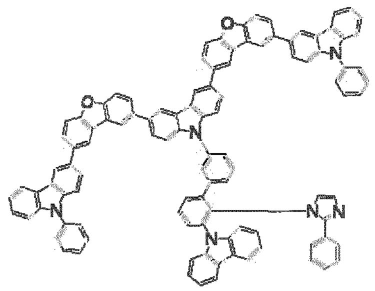

L-126

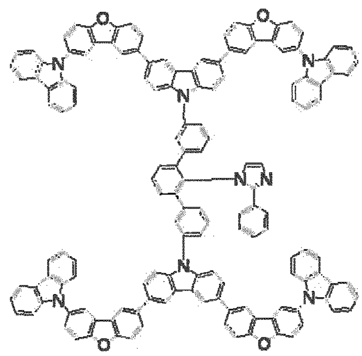

L-127

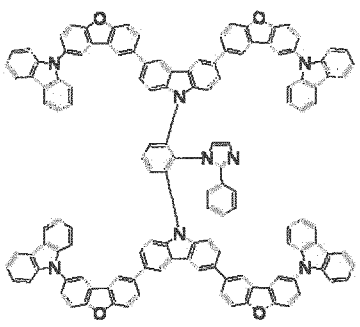

L-128

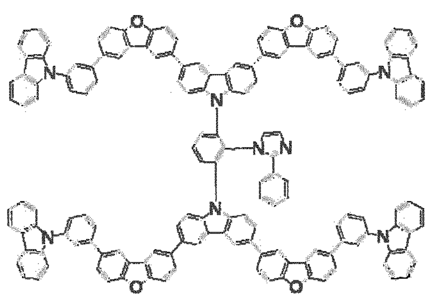

L-129

Organic Metal Complex

Using the imidazole compound manufactured according to the manufacturing method of the imidazole compound according to the exemplary embodiment, an organic metal complex having the imidazole compound as a partial structure can be obtained.

The organic metal complex preferably contains at least one metal selected from metal elements from the eighth group to the eleventh group in the periodic table. The metal is preferably platinum or iridium.

The organic metal complex is exemplified by an organic metal complex including the metal elements from the eighth group to the eleventh group in the periodic table and the imidazole compound represented by the formula (1) as a ligand. Since having the imidazole compound represented by the formula (1) as a ligand, the organic metal complex is usable as a luminescent material exhibiting a sharp emission spectrum and capable of emitting near the short wavelength.

Accordingly, the organic metal complex is particularly useful as a blue-emitting organic-EL-device material. Although examples of the organic metal complex are shown below, the organic metal complex is not limited thereto.

[Formula 11]

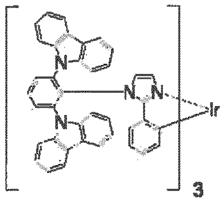

C-1

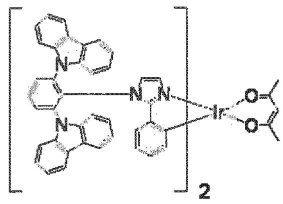

C-2

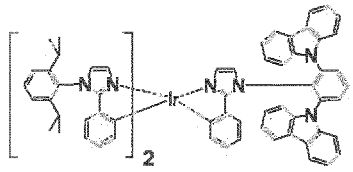

C-3

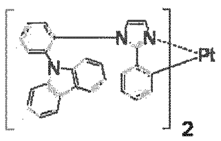

C-4

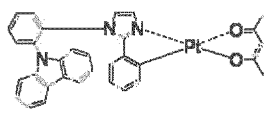

C-5

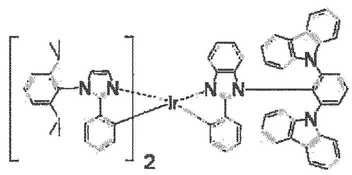

C-6

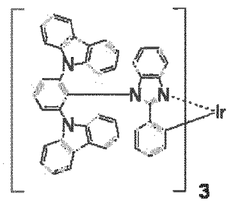

C-7

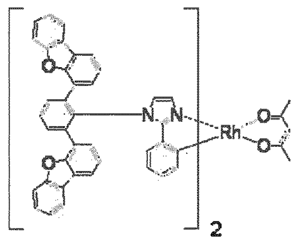

C-8

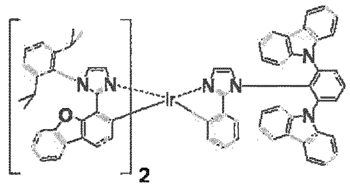

C-9

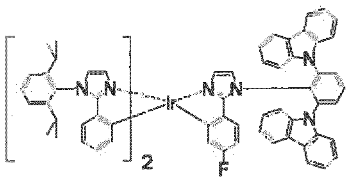

C-10

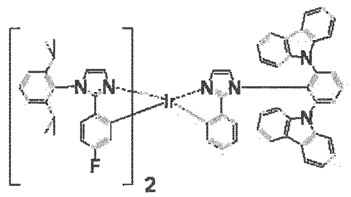

C-11

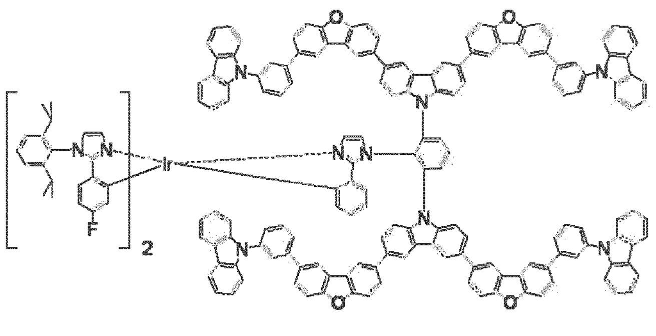

C-12

Organic-Electroluminescence-Device Material

An organic-electroluminescence-device material can be provided using at least one of the imidazole compound manufactured according to the manufacturing method of the imidazole compound according to the exemplary embodiment, the imidazole-based compound and the organic metal complex. Hereinafter, the organic electroluminescence device is referred to as an organic EL device and the organic-electroluminescence-device material is referred to as an organic-EL-device material.

Organic El Device, Display Device, Lighting Device

The following organic EL device may also be regarded as an organic EL device according to another exemplary embodiment of the invention. Specifically, the imidazole compound, which is difficult to manufacture according to a conventional manufacturing method but useful as an organic-EL-device material, can be manufactured according to the above method of the imidazole compound according to the exemplary embodiment. As a result, the following organic EL device containing the imidazole compound difficult to manufacture can be efficiently manufactured.

The organic EL device includes: an anode; a cathode; and an organic compound layer provided between the anode and the cathode and including at least one layer, in which the organic compound layer includes an emitting layer, one of the organic compound layer includes an organic metal complex, and the organic metal complex has an imidazole compound represented by a formula (4) as a partial structure.

[Formula 12]

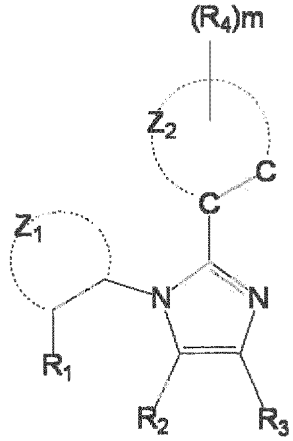

(4)

In the formula (4), $R_1$ is a substituent having a steric parameter (Es) value of-1.70 or less.

$Z_1$ represents a group of atoms necessary for forming a hydrocarbon cyclic group or a heterocyclic group. The hydrocarbon cyclic group or the heterocyclic group formed in $Z_1$ has at least one substituent represented by $R_1$ described below.

$R_2$ and $R_3$ represent a bond, a hydrogen atom or an aromatic hydrocarbon group. $R_2$ and $R_3$ may be mutually bonded to form a five-membered hydrocarbon ring, six-membered hydrocarbon ring, five-membered heterocyclic ring or six-membered heterocyclic ring. These rings may further have a substituent.

$Z_2$ represents a group of atoms necessary for forming a five-membered hydrocarbon ring, six-membered hydrocarbon ring, five-membered heterocyclic ring or six-membered heterocyclic ring together with C-C.

$R_4$ represents a hydrogen atom or a substituent.

m is an integer of 1 to 5.

Since preferable examples of $Z_1$, $Z_2$, $R_1$, $R_2$, $R_3$ and $R_4$ described above are the same as those described in relation to the formula (1), a detailed description of the examples is omitted.

In the formula (4), $R_1$ is a steric parameter (Es) value of -1.70 or less and may be larger than -2.0. $R_1$ is preferably a substituent having the Es value of -2.0 or less, more preferably of -2.5 or less, further preferably of -3.0 or less, particularly preferably of -5.0 or less.

The substituent of $R_1$ preferably has a molecular weight of 43 or more, more preferably of 77 or more, further preferably of 116 or more, still further preferably of 127 or more, particularly preferably of 165 or more.

The organic metal complex having the imidazole compound represented by the formula (4) as a partial structure preferably contains a metal element selected from metal elements from the eighth group to the eleventh group of the periodic table. More preferably, the metal element is either iridium or platinum.

Although the examples of the organic metal complex are shown in the above (C-1) to (C-12), the organic metal complex is not limited thereto.

The organic metal complex is preferably contained in the emitting layer, particularly as an emitting dopant material. This organic EL device is preferably a phosphorescent one.

According to still another exemplary embodiment, the invention also provides a display device or a lighting device including the above organic EL device.

The organic EL device will be described in detail below.

The organic EL device includes: a cathode; an anode; and an organic compound layer between the cathode and the anode, in which the organic compound layer contains at least one layer formed of an organic compound. The organic compound layer may contain an inorganic compound.

In the organic EL device, at least one layer of the organic compound layer contains the organic-EL-device material. The organic compound layer has at least one emitting layer. Accordingly, the organic compound layer may be provided by a single layer of the emitting layer, or may be provided by a laminate of known layers employed in an organic EL device (e.g., a hole injecting layer, a hole transporting layer, an electron injecting layer and an electron transporting layer) through the emitting layer.

Examples of a structure of the multilayered organic EL device include:

(a) anode / hole injecting·transporting layer / emitting layer / cathode;
(b) anode / emitting layer / electron injecting·transporting layer / cathode;
(c) anode / hole injecting·transporting layer / emitting layer / electron injecting·transporting layer / cathode; and
(d) anode / hole injecting·transporting layer / emitting layer / hole blocking layer / electron injecting·transporting layer / cathode.

It should be noted that the "hole injecting·transporting layer" herein means "at least one of hole injecting layer and hole transporting layer" while "electron injecting·transporting layer" herein means "at least one of electron injecting layer and electron transporting layer."

The organic-EL-device material containing the imidazole compound or the imidazole-based compound is usable as a host of the emitting layer. The organic-EL-device material containing the organic metal complex is also usable as a dopant of the emitting layer.

In the latter case, since the organic metal complex exhibits a sharp emission spectrum and emits near the short wavelength, the organic metal complex is particularly useful for forming a blue emitting layer.

A display device and a lighting device capable of displaying in color can be formed by juxtaposing an organic EL device having a red emitting layer and an organic EL device having a green emitting layer as well as the organic EL device having the blue emitting layer.

Alternatively, a red emitting layer and a green emitting layer may be laminated on the blue emitting layer to form a white-emitting organic EL device. In this case, the white-emitting organic EL device preferably has a so-called tandem-type device structure. The white-emitting organic EL device is also applicable to the display device and the lighting device.

In the organic EL device, in addition to the above organic-EL-device material, any material selected from known materials used in a typical organic EL device is usable.

## Second Exemplary Embodiment

A second exemplary embodiment of the invention will be described in detail below.

In the description of the second exemplary embodiment, the same contents and components as those in the first exemplary embodiment are denoted by the same reference numerals or names to simplify or omit description of the contents and the components.

### Imidazole Compound

The imidazole compound according to the second exemplary embodiment is represented by the formula (1).

In the formula (1), $Z_1$ represents a group of atoms necessary for forming a hydrocarbon cyclic group or a heterocyclic group. The hydrocarbon cyclic group or the heterocyclic group has at least one substituent represented by $R_1$ described below. The number of the substituent is preferably 1, 2, 3, 4 or 5. When the number of $R_1$ is plural, $R_1$ may be mutually the same or different. Among the imidazole compound represented by the formula (1), an imidazole compound having $R_1$ at each of ortho positions of the hydrocarbon cyclic group or the heterocyclic group is preferable.

In the formula (1), $R_1$ is a substituent having a steric parameter (Es) value of -2.0 or less, preferably of -2.5 or less, more preferably of -3.0 or less, further preferably of -5.0 or less.

The Es value is a steric parameter induced from chemical reactivity. It can be said that the smaller the Es value is, the

more sterically bulky the substituent is. Accordingly, the smaller Es value is preferable.

The Es value in the exemplary embodiment is a value that is represented as zero for a hydrogen atom.

The substituent having the Es value of -2.0 or less is exemplified by $-CF_3$ (a trifluoromethyl group), a 9-H-fluorene group (an Es value =-2.34) and a carbazolyl group. The substituent having the Es value of -2.5 or less is exemplified by -t-$C_4H_9$ (a tert-butyl group). The substituent having the Es value of -3.0 or less is exemplified by $-CH(C_2H_5)_2$ (a 3-(n-pentyl) group), $-CHBr_2$ (a dibromomethyl group), $-CCl_3$ (a trichloromethyl group) and $-CBr_3$ (a tribromomethyl group). The substituent having the Es value of -5.0 or less is exemplified by $-C(C_6H_5)_3$ (a triphenylmethyl group). These substituents may be further substituted by a later-described substitutent Y. It is speculated that the Es value of the carbazolyl group falls within a range of -2.0 to -2.5 since the 9-H-fluorene group and the carbazolyl group are similar in structure.

In the formula (1), in addition to falling within the range of the Es value, $R_1$ is preferably a substituent having a molecular weight of 43 or more, more preferably of 77 or more, further preferably of 116 or more, still further preferably of 127 or more, still further preferably of 166 or more. The substituent having a molecular weight of 43 or more is exemplified by an isopropyl group (molecular weight: 43). The substituent having a molecular weight of 77 or more is exemplified by a phenyl group (molecular weight: 77). The substituent having a molecular weight of 116 or more is exemplified by an indole group (molecular weight: 116). The substituent having a molecular weight of 127 or more is exemplified by a naphthyl group (molecular weight: 127). The substituent having a molecular weight of 166 or more is exemplified by a carbazole group (molecular weight: 166).

Hydrocarbon Cyclic Group

As described above, in the formula (1), $Z_1$ represents a group of atoms necessary for forming a hydrocarbon cyclic group. The hydrocarbon cyclic group is exemplified by the same cycloalkyl group or aryl group (aromatic cyclic group) as described in the first exemplary embodiment. These groups may have the later-described substituent Y.

Heterocyclic Group

As described above, in the formula (1), $Z_1$ represents a group of atoms necessary for forming a heterocyclic group. The heterocyclic group is exemplified by the same aliphatic heterocyclic group and aromatic heterocyclic group as described in the first exemplary embodiment.

The hydrocarbon cyclic group or heterocyclic group may further have a substituent in addition to the substituent represented by $R_1$. Hereinafter, this substituent is referred to as the substituent Y.

Examples of the substituent Y are the same as those of the substituent represented by $R_1$ in the first exemplary embodiment.

In the formula (1), $R_2$ and $R_3$ represent a bond, a hydrogen atom or an aromatic hydrocarbon group. $R_2$ and $R_3$ may be mutually bonded to form a five-membered hydrocarbon ring, six-membered hydrocarbon ring, five-membered heterocyclic ring or six-membered heterocyclic ring. Further, these rings may have the substituent Y.

Examples of the five- or six-membered hydrocarbon ring and the five- or six-membered heterocyclic ring are the same as those in the first exemplary embodiment.

In the formula (1), $Z_2$ represents a group of atoms necessary for forming a five-membered hydrocarbon ring, six-membered hydrocarbon ring, five-membered heterocyclic ring or six-membered heterocyclic ring together with C-C. The five-membered hydrocarbon ring, six-membered hydrocarbon ring, five-membered heterocyclic ring or six-membered heterocyclic ring are the same as those described in relation to $R_2$ and $R_3$.

In the formula (1), $R_4$ represents a hydrogen atom or a substituent. The substituent is exemplified by the substituent Y.

In the formula (1), m is an integer of 1 to 5. When the number of $R_4$ is 2 or more, $R_4$ may be mutually the same or different.

In the formula (1), the hydrocarbon cyclic group formed by $Z_1$ is preferably an aryl group, more preferably a phenyl group.

Further, second and sixth positions of the phenyl group are preferably substituted by $R_1$.

Examples of specific structures of the imidazole compound according to the exemplary embodiment are as follows. However, the invention is not limited to the imidazole compound having these structures.

[Formula 13]

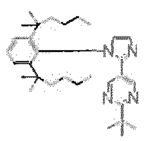
L-1

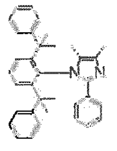
L-2

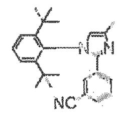
L-3

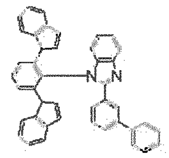
L-4

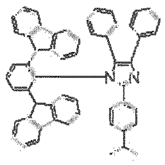
L-5

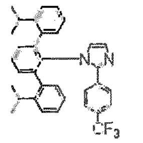
L-6

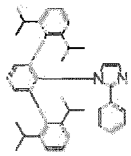
L-7

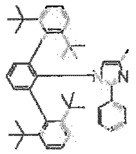
L-8

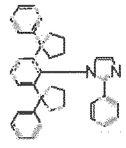
L-9

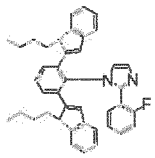
L-10

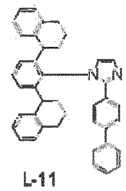
L-11

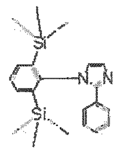
L-12

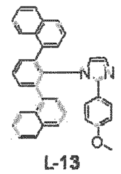
L-13

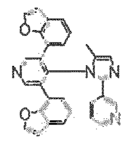
L-14

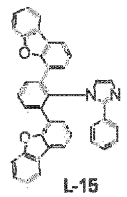
L-15

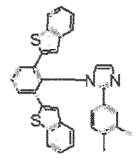
L-16

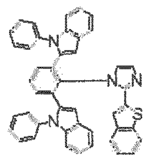
L-17

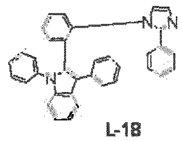
L-18

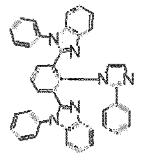
L-19

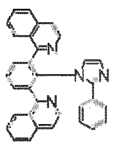
L-20

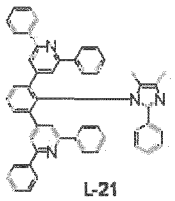
L-21

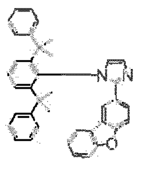
L-22

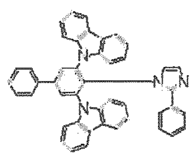
L-23

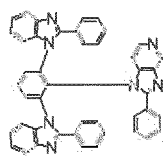
L-24

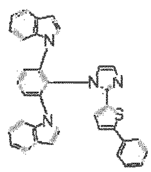
L-25

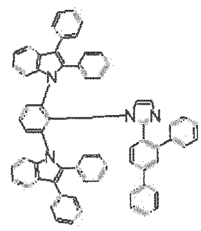
L-26

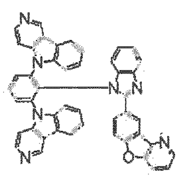
L-27

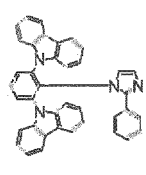
L-28

[Formula 14]

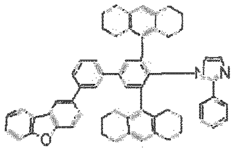
L-29

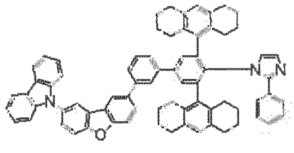
L-30

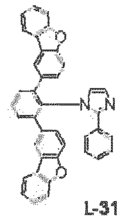
L-31

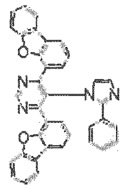
L-32

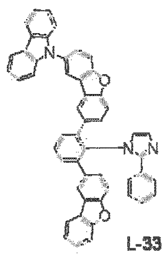
L-33

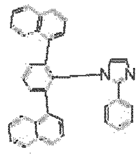
L-34

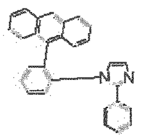
L-35

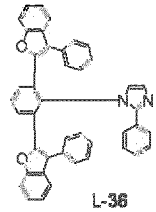
L-36

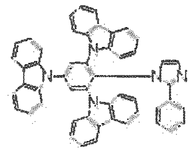
L-37

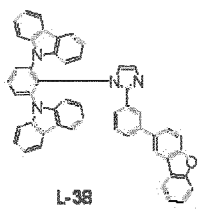
L-38

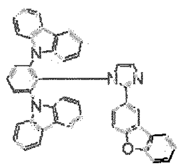
L-39

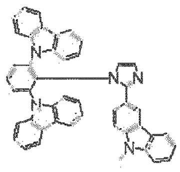
L-40

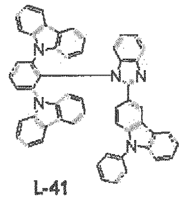
L-41

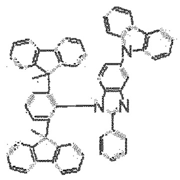
L-42

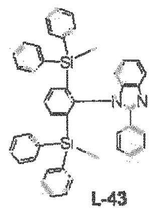
L-43

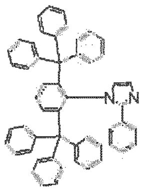
L-44

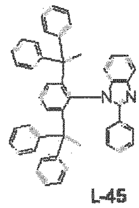
L-45

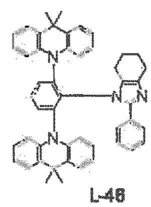
L-46

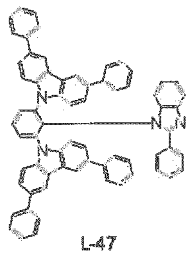
L-47

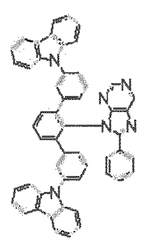
L-48

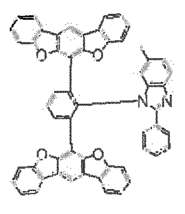
L-49

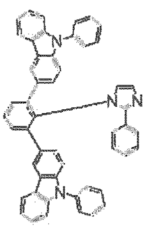
L-50

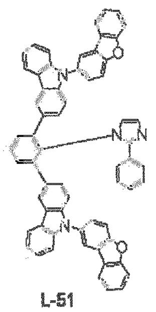
L-51

[Formula 15]

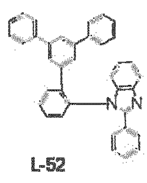
L-52
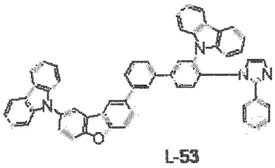
L-53
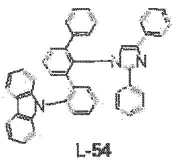
L-54
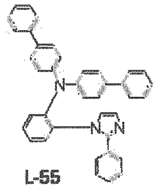
L-55
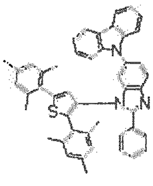
L-56
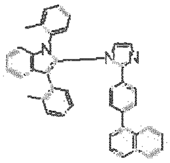
L-57
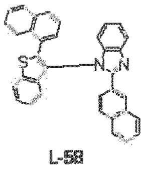
L-58
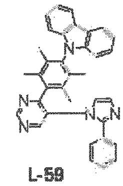
L-59
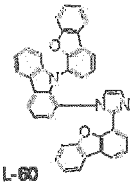
L-60
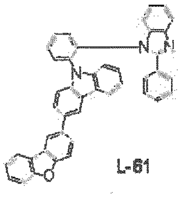
L-61
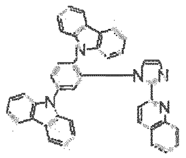
L-62
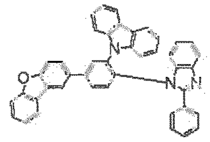
L-63
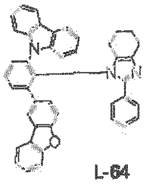
L-64
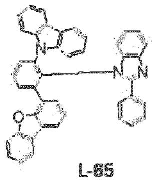
L-65
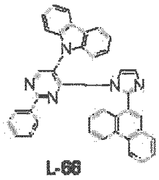
L-66
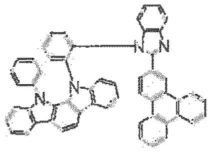
L-67
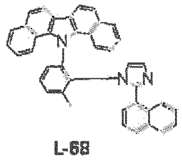
L-68
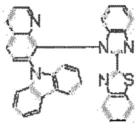
L-69
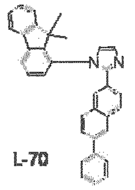
L-70
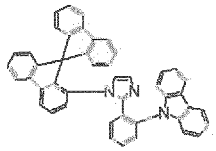
L-71
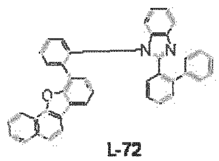
L-72
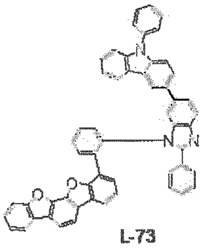
L-73
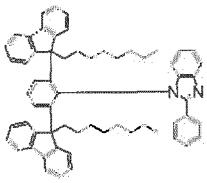
L-74
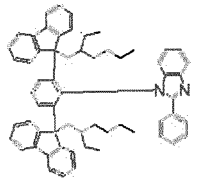
L-75

[Formula 16]

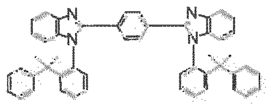
L-76

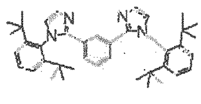
L-77

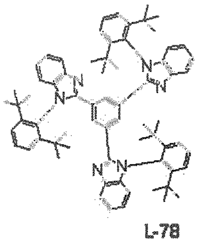
L-78

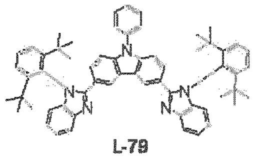
L-79

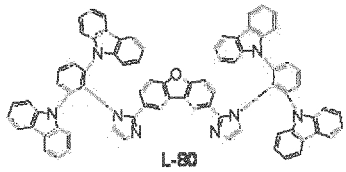
L-80

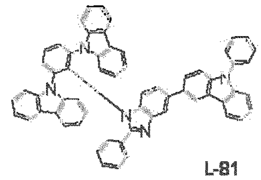
L-81

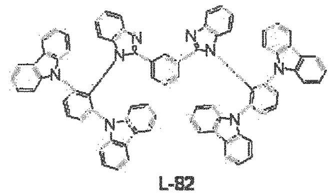
L-82

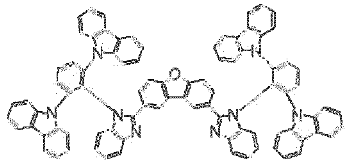
L-83

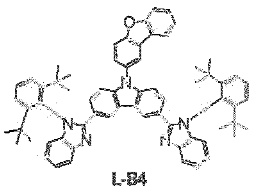
L-84

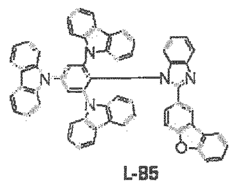
L-85

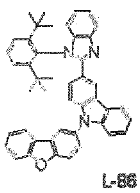
L-86

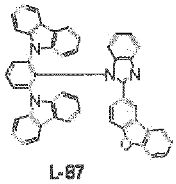
L-87

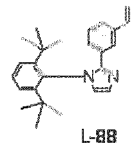
L-88

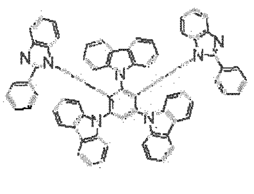
L-89

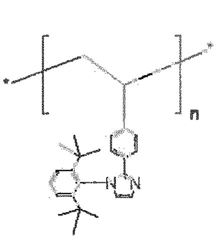
L-90

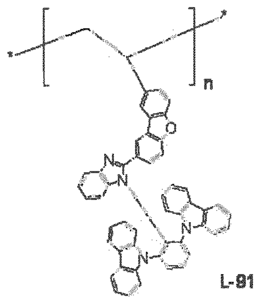
L-91

Manufacturing Method of Imidazole Compound

The imidazole compound according to the second exemplary embodiment is manufactured according to a method described below.

The imidazole compound according to the second exemplary embodiment can be manufactured by reacting compounds represented by the following formulae (2) and (3) in an organic solvent, as needed, in the presence of at least one of an inorganic basic compound and an organic basic compound and in the presence of a metal catalyst (hereinafter, this reaction is referred to as Reaction 2).

[Formula 17]

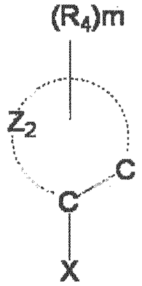

(2)

In the formula (2), X represents a halogen atom and $Z_2$, $R_4$ and m represent the same as those in the formula (1).

[Formula 18]

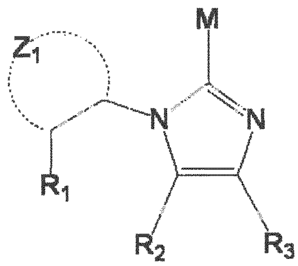

(3)

In the formula (3), M represents a boron atom, a magnesium atom, a silicon atom, a stannum atom and a zinc atom which are substituted or unsubstituted. $Z_1$, $R_1$, $R_2$ and $R_3$ represent the same as those in the formula (1).

Examples of the inorganic basic compound, the organic basic compound and the metal catalyst which are used as needed for the Reaction 2 are the same as those in the first exemplary embodiment..

Preferable examples of the organic solvent used for the Reaction 2 are an ether solvent, an aliphatic hydrocarbon solvent and an aromatic hydrocarbon solvent.

In the reaction system of the Reaction 2, the smaller volume of the ether solvent having at most 5 carbon atoms is preferable. Specifically, for performing the Reaction 2, in a reaction system, a mole number $N_{f2}$ [mol] of the compound represented by the formula (2) and a total volume $V_A$ [liter] of the ether solvent having at most 5 carbon atoms preferably satisfy the relationship of the following numerical formula (1), more preferably of the following numerical formula (2), further preferably of the following numerical formula (3). When plural kinds of the ether solvent having at most 5 carbon atoms are contained in the reaction system, $V_A$ represents a total volume of the plural kinds of the ether solvent.

[Numerical Formula 7]

$$V_A/N_{f2} \leqq 3 \quad \cdots(1)$$

[Numerical Formula 8]

$$V_A/N_{f2} \leq 2 \qquad (2)$$

[Numerical Formula 9]

$$V_A/N_{f2} \leq 1 \qquad (3)$$

Any one of the numerical formulae (1) to (3) is satisfied by adjusting the volume of the ether solvent having at most 5 carbon atoms which is added in preparation for the Reaction 2. When the following numerical formula (4) is

satisfied at the time of performing the Reaction 2 due to solubility of a reagent used for the Reaction 2 and steps prior to the Reaction 2, a solvent removal treatment to the reaction system is conducted, thereby adjusting the volume of the ether solvent having at most 5 carbon atoms so as to satisfy any one of the numerical formulae (1) to (3).

[Numerical Formula 10]

$$V_A/N_{f2}>3 \cdots(4)$$

Examples of the ether solvent having at most 5 carbon atoms and the solvent removal treatment are the same as those in the first exemplary embodiment.

As compared with a case where only the ether solvent having at most 5 carbon atoms is contained in the reaction system, a case where at least one of an ether solvent having at least 6 carbon atoms, an aliphatic hydrocarbon solvent having at least 7 carbon atoms and an aromatic hydrocarbon solvent is contained in the reaction system is more preferable.

In this case, for performing the Reaction 2, a total volume $V_B$ [liter] of at least one solvent selected from the ether solvent having at least 6 carbon atoms, the aliphatic hydrocarbon solvent having at least 7 carbon atoms and the aromatic hydrocarbon solvent in the reaction system and a mole number $N_{f2}$ [mol] of the compound represented by the formula (2) preferably satisfy the relationship of the following numerical formula (5), more preferably of the following numerical formula (6).

[Numerical Formula 11]

$$V_B/N_{f2}\geq 0.1 \qquad (5)$$

[Numerical Formula 12]

$$10\geq V_B/N_{f2}\geq 0.1 \qquad (6)$$

Examples of the ether solvent having at least 6 carbon atoms, the aliphatic hydrocarbon having at least 7 carbon atoms and the aromatic hydrocarbon solvent are the same as those in the first exemplary embodiment

By performing the Reaction 2 under these conditions, the imidazole compound including a hydrocarbon cyclic group or a heterocyclic group containing a sterically bulky substituent $R_1$ in $Z_1$, which is incapable of being synthesized according to a conventionally disclosed synthesis method, can be synthesized.

Imidazole-based Compound

Examples of an imidazole-based compound according to the second exemplary embodiment are the same as those in the first exemplary embodiment. However, the imidazole-based compound is not limited thereto.

Organic Metal Complex

The organic metal complex according to the second exemplary embodiment has the imidazole compound represented by the formula (1) as a partial structure.

The organic metal complex according to the second exemplary embodiment preferably contains at least one metal selected from metal elements from the eighth group to the eleventh group in the periodic table. The metal is preferably platinum or iridium.

The organic metal complex according to the exemplary embodiment is exemplified by an organic metal complex including the metal elements from the eighth group to the eleventh group in the periodic table and the imidazole compound represented by the formula (1) as a ligand. The organic metal complex of the invention can be synthesized by a known method.

Since having the imidazole compound represented by the formula (1) as a ligand, the organic metal complex according to the exemplary embodiment is usable as a luminescent material exhibiting a sharp emission spectrum and capable of emitting near the short wavelength. Accordingly, the organic metal complex is particularly useful as a blue-emitting organic-EL-device material.

Examples of the organic metal complex having the imidazole compound as a partial structure in the second exemplary embodiment are the same as those in the first exemplary embodiment. However, the organic metal complex

is not limited thereto.

Organic-Electroluminescence-Device Material

An organic-electroluminescence-device material according to the second exemplary embodiment preferably contains at least one of the imidazole compound represented by the formula (1), the organic compound and the organic metal complex.

Organic EL Device, Display Device, Lighting Device

Examples of a layer structure of the organic EL device according to the second exemplary embodiment are the same as those in the first exemplary embodiment.

In the same manner as in the first exemplary embodiment, the organic-EL-device material containing the imidazole compound or the imidazole-based compound according to the second exemplary embodiment is usable as a host of the emitting layer, and the organic-EL-device material containing the organic metal complex according to the second exemplary embodiment is usable as a dopant of the emitting layer. Advantageous effects obtained by the above arrangement are the same as those in the first exemplary embodiment.

In the organic EL device according to the second exemplary embodiment, in addition to the organic-EL-device material according to the second exemplary embodiment, any material selected from known materials used in a typical organic EL device is usable.

Examples

Examples of the invention will be described below. However, the invention is not limited by these Examples.

Example 1: Synthesis of Imidazole Compound (Compound 3)

Synthesis Example 1: Synthesis of Compound 1

[Formula 19]

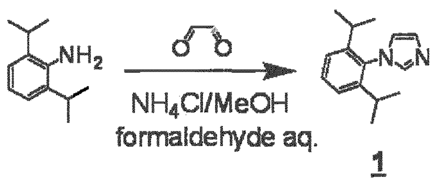


1

To a three-necked flask, methanol (100 ml), a glyoxal aqueous solution (40 mass%) (11.4 ml, 100 mmol) and 2,6-diisopropylaniline (17.73 g, 100 mmol) were put and stirred for 16 hours at the room temperature. Subsequently, methanol (400 ml), ammonium chloride (6.42 g, 120 mmol) and a formaldehyde aqueous solution (37 mass%) (16.2 ml, 200 mmol) were added and refluxed for eight hours.
After the reaction, the mixture was concentrated by an evaporator and adjusted to pH10 with an aqueous sodium hydroxide. A sample was transferred to a separating funnel and extracted with dichloromethane. After dried over anhydrous magnesium sulfate, the extract was filtrated and concentrated. The obtained product was refined by silica-gel chromatography (dichloromethane:ethyl acetate=9:1 (volume ratio)) and was further recrystallized in hexane to obtain a white solid (a compound 1).
The compound 1 was identified according to $^1$H-NMR and FD-MS.
Yield: 9.2 g
Yield Rate: 40%

Synthesis Example 2: Synthesis of Compound 2

[Formula 20]

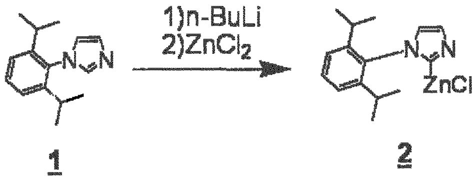


1 2

Under nitrogen atmosphere, to a three-necked flask, the compound 1 (27.4 g, 60 mmol) and tetrahydrofuran (60ml) were put and dissolved. The obtained solution was cooled to 0 degree C. An n-BuLi-hexane solution (35.9 ml, mole number of n-BuLi in the solution: 60 mmol, molarity (mole number of n-BuLi/solution amount): 1.67M) was added thereto over 10 minutes and stirred for 30 minutes at 0 degree C. Next, a solution of zinc chloride (13.6 g, 100 mmol) dissolved in tetrahydrofuran (100 ml) was added thereto over 10 minutes. Subsequently, the obtained solution was recovered to the room temperature. As a solvent of the n-BuLi-hexane solution, n-hexane was used. The same applies to other Examples and Comparatives described later.
Without being refined or the like, a compound 2 was used for the following reaction.

Synthesis Example 3: Synthesis of Compound 3

[Formula 21]

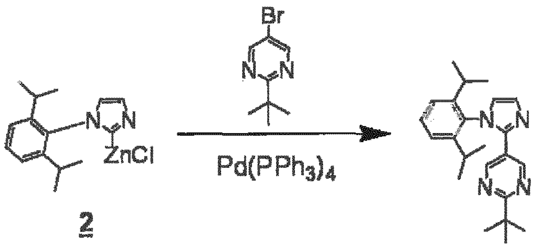


2 3

After preparing the compound 2 in Synthesis Example 2, the compound 2 was put into a three-necked flask, to which an oil-sealed rotary pump was connected through a solvent trap that is cooled using a solvent of dry ice/acetone. The three-necked flask was heated to about 40 degrees C under reduced pressure to remove the solvent (150 ml) from the reaction system (a solvent removal step). A removal amount of the solvent herein is an amount collected by the solvent trap.
Subsequently, nitrogen was put into the reaction system to return to the normal pressure. 2-tert-butyl-5-Bromopyrimidine (10.8 g, 50 mmol) and $Pd(PPh_3)_4$ (2.89 g, 2.5 mmol) were put into the reaction system and reacted at 90 degrees C for 16 hours under nitrogen atmosphere.
A relationship of the solvent amounts in the reaction system in Synthesis Example 3 is shown in Table 1.
It should be noted that n-butane was formed by changing n-BuLi (molecular weight MW=64.06) in the reaction of Synthesis Example 2. Specifically, n-BuLi (60 mmol) was changed to n-butane (60 mmol, 5.8ml). A volume of n-butane was calculated based on a molecular weight of n-butane (MW=58.12) and a specific gravity of n-butane (0.60).
The volume of n-hexane was calculated as follows. Firstly, a solution mass (24.4 g) was calculated from a specific gravity (0.68) of the n-BuLi-hexane solution. Next, a mass of n-BuLi (3.84 g) was subtracted from the solution mass to calculate a mass of n-hexane (20.56 g). The mass of n-hexane was converted to the volume using a specific gravity (0.66) of n-hexane.
The same calculation applies to other Examples and Comparatives described later.
Table 1

| | Example 1 | Example 2 | Example 3 | Example 4 | Comp. 1 | Comp. 2 | Comp. 3 | Comp. 4 |
|---|---|---|---|---|---|---|---|---|
| | Synthesis Ex.3 | Synthesis Ex. 4 | Synthesis Ex. 5 | Synthesis Ex. 10 | Synthesis Ex. 11 | Synthesis Ex. 12 | Synthesis Ex. 13 | Synthesis Ex. 14 |
| THF[ml] | 60.0 | 125.0 | 140.0 | 72.0 | 60.0 | 125.0 | 140.0 | 72.0 |
| n-hexane [ml] | 31.2 | 64.9 | 36.4 | 6.2 | 31.2 | 64.9 | 36.4 | 6.2 |
| n-butane [ml] | 5.8 | 12.1 | 6.8 | 1.2 | 5.8 | 12.1 | 6.8 | 1.2 |
| THF ($ZnCl_2$) [ml] | 100.0 | 208.0 | 100.0 | 15.0 | 100.0 | 208.0 | 100.0 | 15.0 |
| Solvent total [ml] (before solvent removal step) | 197.0 | 410.0 | 283.1 | 94.4 | 197.0 | 410.0 | 283.1 | 94.4 |
| Solvent removal [ml] | 150.0 | 310.0 | 250.0 | 90.0 | 0.0 | 0.0 | 0.0 | 0.0 |
| Solvent in flask [ml] (after solvent removal step) | 47.0 | 100.0 | 33.1 | 4.4 | 197.0 | 410.0 | 283.1 | 94.4 |
| THF [ml] (=$V_A$) | ≦ 47.0 | ≦ 100.0 | ≦ 33.1 | ≦ 4.4 | 160.0 | 333.0 | 240.0 | 87.0 |
| toluene [ml] (= $V_B$) (addition of toluene) | 0.0 | 104.0 | 50.0 | 30.0 | 0.0 | 0.0 | 0.0 | 0.0 |
| Compound of Formula (2) [mmol] (=$Nf_2$) | 50.0 | 104.0 | 50.0 | 10.0 | 50.0 | 104.0 | 50.0 | 10.0 |
| Calculation by Formula $V_A/Nf_2$ | ≦ 0.94 | ≦ 0.96 | ≦0.66 | ≦0.44 | 3.20 | 3.20 | 4.80 | 8.70 |
| Calculation by Formula $V_B/Nf_2$ | 0.00 | 1.00 | 1.00 | 3.00 | 0.00 | 0.00 | 0.00 | 0.00 |
| TH F : tetrahydrofuran<br>$ZnCl_2$: Zinc chloride<br>Compound of Formula (2):2-tert-butyl-5-Bromopyrimidine, iodobenzene, 2-Bromodibenzofuran | | | | | | | | |

As shown in Table 1, since the total amount of the solvent left in the flask after the solvent removal step was 47.0 ml, it can be judged that the total volume $V_A$ [liter] of tetrahydrofuran (i.e., the ether solvent having at most 5 carbon atoms) was 47.0 ml or less.

Accordingly, a relationship between the total volume $V_A$ [liter] of tetrahydrofuran (i.e., the ether solvent having at most 5 carbon atoms) and the mole number $N_{f2}$ [mol] of 2-tert-butyl-5-Bromopyrimidine is represented below as shown in Table 1.

$$V_A/N_{f2} \leq 0.94$$

The relationship of the numerical formula (3) was satisfied.

After the reaction, a small amount of water was added to the sample to deactivate the reaction. The mixture was diluted with dichloromethane (200ml). An aqueous solution of tetrasodium ethylenediaminetetraacetate dihydrate (62.43 g, 150 mmol) was added to the diluted mixture and stirred in a separating funnel. An aqueous sodium hydroxide was further added thereto to adjust an aqueous phase to pH10 or more. A dichloromethane phase was collected. The aqueous phase was extracted with dichloromethane several times. The extract was dried over anhydrous magnesium sulfate, filtrated and concentrated. The obtained product was refined by silica-gel chromatography (dichloromethane: acetone=95:5 (volume ratio)) and was further recrystallized in a mixture solvent of hexane/ethyl acetate to obtain a white solid (a compound 3).

The compound 3 was identified according to $^1$H-NMR and FD-MS.

Yield: 15.2 g

Yield Rate: 84%

Example 2: Synthesis of Imidazole Compound (Compound 4)

Synthesis Example 4: Synthesis of Compound 4

[Formula 22]

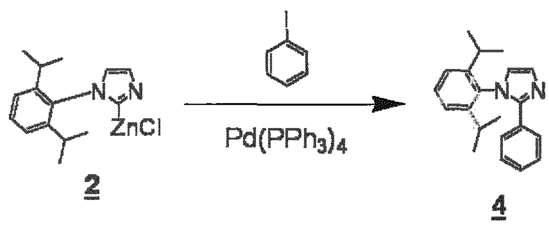


In the same procedure as in Synthesis Example 2, the compound 2 was prepared from a solution containing the compound 1 (28.5 g, 125 mmol), tetrahydrofuran (125ml), the n-BuLi-hexane solution (74.9 ml, mole number of n-BuLi in the solution: 125 mmol, molarity (mole number of n-BuLi/solution amount): 1.67M) and zinc chloride (28.4 g, 208 mmol) dissolved in tetrahydrofuran (208 ml). After preparing the compound 2, the compound 2 was put into a three-necked flask, to which an oil-sealed rotary pump was connected through a solvent trap that is cooled using a solvent of dry ice/acetone. The three-necked flask was heated to about 40 degrees C under reduced pressure to remove the solvent (310 ml) from the reaction system (the solvent removal step). A removal amount of the solvent herein is an amount collected by the solvent trap.

Subsequently, nitrogen was put into the reaction system to return to the normal pressure. Toluene (104 ml) was put into the reaction system (toluene addition step). Next, iodobenzene (21.3g, 104 mmol) and $Pd(PPh_3)_4$ (2.89 g, 2.5 mmol) were added thereto and reacted at 120 degrees C for 16 hours under nitrogen atmosphere.

A relationship of the solvent amounts in the reaction system in Synthesis Example 4 is shown in Table 1. Herein, a relationship between the total volume $V_A$ [liter] of tetrahydrofuran (i.e., the ether solvent having at most 5 carbon atoms) and the mole number $N_{f2}$ [mol] of iodobenzene is represented below according the same calculation as in Synthesis Example 3 as shown in Table 1.

$$V_A/N_{f2} \leq 0.96$$

The relationship of the numerical formula (3) was satisfied.
Further, a relationship between a volume $V_B$ [liter] of toluene (i.e., an aromatic hydrocarbon solvent having 7 carbon atoms) and a mole number $N_{f2}$ [mol] of iodobenzene is represented below as shown in Table 1.

$$V_B/N_{f2}=1$$

The relationship of the numerical formula (5) was satisfied.
There is a possibility that n-hexane (i.e., an aliphatic hydrocarbon having 6 carbon atoms) is contained in the reaction system even after the solvent removal step in Synthesis Example 4. However, since the volume of n-hexane after the solvent removal step is less than the volume of toluene subsequently added, the relationship of the numerical formula (5) is still satisfied even in consideration of n-hexane. The same applies to Examples 3 and 4 described later.
Accordingly, Synthesis Example 4 was conducted under the reaction condition of satisfying the relationships of the numerical formulae (3) and (5).

After the reaction, a small amount of water was added to the sample to deactivate the reaction. The mixture was diluted with dichloromethane (300 ml). An aqueous solution of tetrasodium ethylenediaminetetraacetate dihydrate (129.9 g, 312 mmol) was added to the diluted mixture and stirred in a separating funnel. An aqueous sodium hydroxide was further added thereto to adjust an aqueous phase to pH10 or more. A dichloromethane phase was collected. The aqueous phase was extracted with dichloromethane several times. The extract was dried over anhydrous magnesium sulfate, filtrated and concentrated. The obtained product was refined by silica-gel chromatography (toluene:ethyl acetate=95:5 (volume ratio)) and was further recrystallized in a mixture solvent of hexane/ethyl acetate to obtain a white solid. The compound 4 was identified according to $^1$H-NMR and FD-MS.
Yield: 24.7 g
Yield Rate: 78%

Example 3: Synthesis of Imidazole Compound (Compound 5)

Synthesis Example 5: Synthesis of Compound 5

[Formula 23]

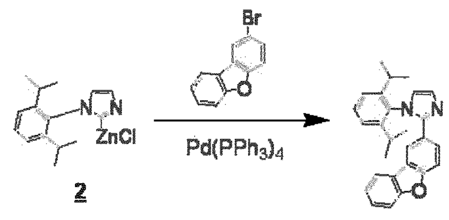


2

5

In the same procedure as in Synthesis Example 2, the compound 2 was prepared from a solution containing the compound 1 (15.98 g, 70 mmol), tetrahydrofuran (140 ml), the n-BuLi-hexane solution (41.9 ml, mole number of n-BuLi in the solution: 70 mmol, molarity (mole number of n-BuLi/solution amount): 1.67M) and zinc chloride (13.6 g, 100 mmol) dissolved in tetrahydrofuran (100 ml). After preparing the compound 2, the compound 2 was put into a three-necked flask, to which an oil-sealed rotary pump was connected through a solvent trap that is cooled using a solvent of dry ice/acetone. The three-necked flask was heated to about 40 degrees C under reduced pressure to remove the solvent (250 ml) from the reaction system (the solvent removal step). A removal amount of the solvent herein is an amount collected by the solvent trap.
Subsequently, nitrogen was put into the reaction system to return to the normal pressure. Toluene (50 ml) was put into the reaction system (a toluene addition step). Next, 2-Bromodibenzofuran (12.35 g, 50 mmol) and $Pd(PPh_3)_4$ (2.89 g, 2.5 mmol) were added thereto and reacted at 120 degrees C for 18 hours under nitrogen atmosphere.

A relationship of the solvent amounts in the reaction system in Synthesis Example 5 is shown in Table 1.
A relationship between the total volume $V_A$ [liter] of tetrahydrofuran (i.e., the ether solvent having at most 5 carbon atoms) and the mole number $N_{f2}$ [mol] of 2-Bromodibenzofuran is represented below according the same calculation as in Synthesis Example 3 as shown in Table 1.

$$V_A/N_{f2} \leq 0.66$$

The relationship of the numerical formula (3) was satisfied.
Further, a relationship between a volume $V_B$ [liter] of toluene and a mole number $N_{f2}$ [mol] of 2-Bromodibenzofuran is represented below as shown in Table 1.

$$V_B/N_{f2}=1$$

The relationship of the numerical formula (5) was satisfied.
Accordingly, Synthesis Example 5 was conducted under the reaction condition of satisfying the relationships of the numerical formulae (3) and (5).

After the reaction, a small amount of water was added to the sample to deactivate the reaction. The mixture was diluted with dichloromethane (300 ml). An aqueous solution of tetrasodium ethylenediaminetetraacetate dihydrate (62.4 g, 150 mmol) was added to the diluted mixture and stirred in a separating funnel. An aqueous sodium hydroxide was further added thereto to adjust an aqueous phase to pH10 or more. A dichloromethane phase was collected. The aqueous phase was extracted with dichloromethane several times. The extract was dried over anhydrous magnesium sulfate, filtrated and concentrated. The obtained product was refined by silica-gel chromatography (dichloromethane: ethyl acetate=95:5 (volume ratio)) and was further recrystallized in a mixture solvent of hexane/ethyl acetate to obtain a white solid (a compound 5).
The compound 5 was identified according to $^1$H-NMR and FD-MS.
Yield: 15.8 g
Yield Rate: 80%

Example 4: Synthesis of Imidazole Compound (Compound 9)

Synthesis Example 6: Synthesis of Compound (2-Fluoro-1,3-diiodobenzene)

[Formula 24]

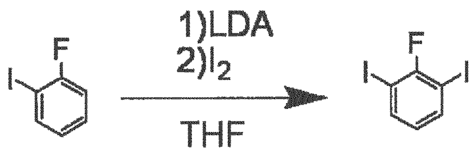


Under nitrogen atmosphere, to a three-necked flask, 2-Fluoroiodobenzene (75.0 g, 338 mmol) and tetrahydrofuran (676 ml) were put and cooled to -70 degrees C, to which a solution of lithium diisopropylamido-tetrahydrofuran was dropped over 20 minutes. The solution of lithium diisopropylamido/tetrahydrofuran was prepared in advance from diisopropylamine (41.0 g, 405.6 mmol), the n-BuLi-hexane solution (228.1ml, mole number of n-BuLi in the solution: 371.8 mmol, molarity (mole number of n-BuLi/solution amount): 1.63M) and tetrahydrofuran (300 ml). After the obtained mixture was stirred for one hour at -70 degrees C, iodine (94.4 g, 371.8 mmol) was added to the mixture. The mixture was slowly recovered to the room temperature and stirred for 12 hours.
After the reaction, a small amount of water was added to the mixture to deactivate the reaction. Subsequently, the obtained mixture was concentrated by an evaporator. An aqueous solution of sodium thiosulfate-sodium hydroxide was added to the mixture to deactivate remaining iodine. A sample was transferred to a separating funnel and extracted with dichloromethane three times. The extract was dried over anhydrous magnesium sulfate, filtrated and concentrated. The obtained product was refined by silica-gel chromatography (hexane) and was further recrystallized in hexane (150ml) at -10 degrees C to obtain a white solid (2-Fluoro-1,3-diiodobenzene).
This compound was identified according to $^1$H-NMR and FD-MS.
Yield: 62.0 g
Yield Rate: 53%

Synthesis Example 7: Synthesis of Compound 6

[Formula 25]

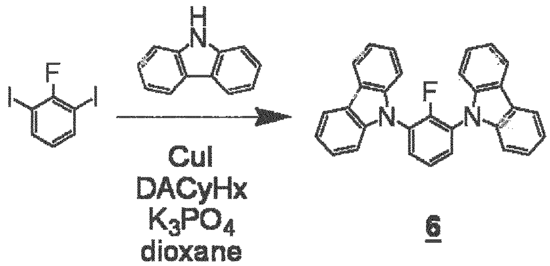


6

Under nitrogen atmosphere, to a three-necked flask, 2-Fluoro-1,3-diiodobenzene (61.9 g, 178 mmol), carbazole (71.4 g, 427.2 mmol), $K_3PO_4$ (151.14 g, 712 mmol), CuI (6.78 g, 35.6 mmol), trans-1,2-diaminocyclohexane (12.8 ml, 106.8 mmol) and 1,4-dioxane (178 ml) were put and refluxed for 16 hours.
After the reaction, the mixture was cooled to the room temperature. Subsequently, a sample was dissolved in toluene (500ml) and filtrated with cerite to separate an inorganic salt. The filtrate was concentrated. Methanol was added to this filtrate to deposit the sample. The sample was subjected to dispersion and washing, collected by filtration and vacuum-dried (50 degrees C for eight hours) to obtain a white solid (a compound 6).
The compound 6 was identified according to $^1$H-NMR and FD-MS.
Yield: 42.57 g
Yield Rate: 56%

Synthesis Example 8: Synthesis of Compound 7

[Formula 26]

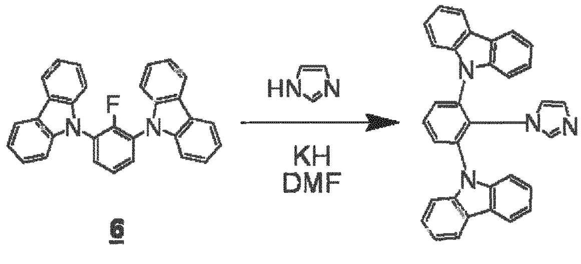


6

7

Under nitrogen atmosphere, to a three-necked flask, potassium hydride (3.0 g, 75 mmol) and N,N-dimethylformamide (50 ml) were put and cooled to 0 degree C. A solution of imidazole (6.81 g, 100 mmol) dissolved in N,N-dimethylformamide (50 ml) was added thereto over 20 minutes while taking care to a generation speed of hydrogen gas. Subsequently, the obtained mixture was stirred for one hour at the room temperature. Next, the compound 6 (21.32 g, 50 mmol) was added thereto and refluxed for six hours.
After the reaction, water (200 ml) was added thereto and the deposited sample was obtained by filtration. The sample was dissolved in dichloromethane, dried over anhydrous magnesium sulfate, filtrated and concentrated. The obtained product was refined by silica-gel chromatography (dichloromethane:ethyl acetate=9:1 (volume ratio)) and was subsequently subjected to dispersion washing in hexane, filtration and vacuum-drying (50 degrees C for eight hours) to obtain a white solid (a compound 7).
The compound 7 was identified according to $^1$H-NMR and FD-MS.
Yield: 17.79 g
Yield Rate: 75%

Synthesis Example 9: Synthesis of Compound 8

[Formula 27]

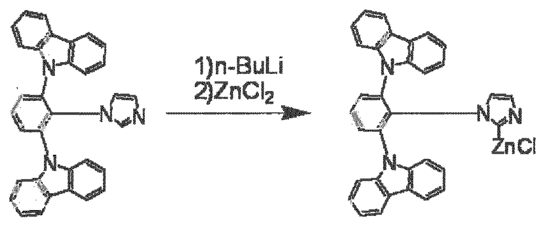


7 8

Under nitrogen atmosphere, to a three-necked flask, the compound 7 (5.69 g, 12 mmol) and tetrahydrofuran (72 ml) were put. Next, the n-BuLi-hexane solution (7.2 ml, mole number of n-BuLi in the solution: 12 mmol, molarity (mole number of n-BuLi/solution amount): 1.67M) was added thereto at the room temperature and stirred for 30 minutes. Next, the solution of zinc chloride (2.04 g, 15 mmol) dissolved in tetrahydrofuran (15 ml) was added thereto over five minutes.
Without being refined or the like, the compound 8 was used for the following reaction.

Synthesis Example 10: Synthesis of Compound 9

[Formula 28]

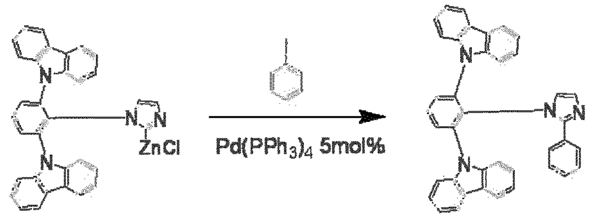


8 9

After synthesizing the compound 8 in Synthesis Example 9, the compound 8 was put into a three-necked flask, to which an oil-sealed rotary pump was connected through a solvent trap that is cooled using a solvent of dry ice/acetone. The three-necked flask was heated to about 40 degrees C under reduced pressure to remove the solvent (90 ml) from the reaction system (the solvent removal step). A removal amount of the solvent herein is an amount collected by the solvent trap.
Subsequently, after cooled to the room temperature, nitrogen was put into the reaction system to return to the normal pressure. Toluene (30 ml) was put into the reaction system (the toluene addition step). Next, iodobenzene (2.04 g, 10 mmol) and $Pd(PPh_3)_4$ (231 mg, 0.2 mmol) were added thereto and reacted at 120 degrees C for 16 hours under nitrogen atmosphere.

A relationship of the solvent amounts in the reaction system in Synthesis Example 10 is shown in Table 1.
As shown in Table 1, since the total amount of the solvent left in the flask after the solvent removal step was 4.4 ml, it can be judged that the total volume $V_A$ [liter] of tetrahydrofuran (i.e., the ether solvent having at most 5 carbon atoms) was 4.4 ml or less.
Accordingly, a relationship between the total volume $V_A$ [liter] of tetrahydrofuran (i.e., the ether solvent having at most 5 carbon atoms) and the mole number $N_{f2}$ [mol] of iodobenzene is represented below as shown in Table 1.

$$V_A/N_{f2} \leq 0.44$$

The relationship of the numerical formula (3) was satisfied.
Further, a relationship between a volume $V_B$ [liter] of toluene and a mole number $N_{f2}$ [mol] of iodobenzene is represented below as shown in Table 1.

$$V_B/N_{f2}=3.0$$

The relationship of the numerical formula (5) was satisfied.
Accordingly, Synthesis Example 10 was conducted under the reaction condition of satisfying the relationships of the numerical formulae (3) and (5).

After the reaction, a small amount of water was added to the sample to deactivate the reaction. The mixture was diluted with dichloromethane (200 ml). An aqueous solution of tetrasodium ethylenediaminetetraacetate dihydrate (8.32 g, 20 mmol) was added to the diluted mixture and stirred in a separating funnel. An aqueous sodium hydroxide was further added thereto to adjust an aqueous phase to pH10 or more. A dichloromethane phase was collected. The aqueous phase was extracted with dichloromethane several times. The extract was dried over anhydrous magnesium sulfate, filtrated and concentrated. The obtained product was refined by silica-gel chromatography (toluene:ethyl acetate=90:10 (volume ratio)) and was subsequently subjected to dispersion washing in methanol to obtain a white solid (a compound 9).
The compound 9 was identified according to $^1$H-NMR and FD-MS. Figs. 1 and 2 show a $^1$H-NMR spectrum of the compound 5.
Yield: 2.3 g
Yield Rate: 42%

Comparative 1: Synthesis of Imidazole Compound (Compound 3)

Synthesis Example 11

[Formula 29]

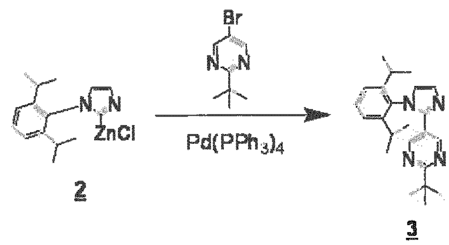


In the same procedure as in Synthesis Example 2, the compound 2 was prepared from a solution containing the compound 1 (27.4 g, 60 mmol), tetrahydrofuran (60 ml), the n-BuLi-hexane solution (35.9 ml, mole number of n-BuLi in the solution: 60 mmol, molarity (mole number of n-BuLi/solution amount): 1.67M) and zinc chloride (13.6 g, 100 mmol) dissolved in tetrahydrofuran (100ml). After the preparation of the compound 2, 2-tert-butyl-5-Bromopyrimidine (10.8 g, 50 mmol) and $Pd(PPh_3)_4$ (2.89 g, 2.5 mmol) were put into the three-necked flask containing the compound 2 and reacted at 90 degrees C for 16 hours under nitrogen atmosphere. In short, in Comparative 1, the solvent removal step was not implemented and toluene was not added.

A relationship of the solvent amounts in the reaction system in Synthesis Example 11 is shown in Table 1.
The relationship between the total volume $V_A$ [liter] of tetrahydrofuran (i.e., the ether solvent having at most 5 carbon atoms) and the mole number $N_{f2}$ [mol] of 2-tert-butyl-5-Bromopyrimidine is represented below according the same calculation as in Synthesis Example 3 as shown in Table 1.

$$V_A/N_{f2}=3.2$$

The relationship of the numerical formula (1) was not satisfied.

After the reaction, a small amount of water was added to the sample to deactivate the reaction. The mixture

was diluted with dichloromethane (200 ml). An aqueous solution of tetrasodium ethylenediaminetetraacetate dihydrate (62.43 g, 150 mmol) was added to the diluted mixture and stirred in a separating funnel. An aqueous sodium hydroxide was further added thereto to adjust an aqueous phase to pH10 or more. A dichloromethane phase was collected. The aqueous phase was extracted with dichloromethane several times. The extract was dried with anhydrous magnesium sulfate, filtrated and concentrated. The obtained product was refined by silica-gel chromatography (dichloromethane: acetone=95:5 (volume ratio)) to obtain a white solid (the compound 3).
The compound 3 was identified according to $^1$H-NMR and FD-MS.
Yield: 0.72 g
Yield Rate: 4%

Comparative 2: Synthesis of Imidazole Compound (Compound 4)

Synthesis Example 12

[Formula 30]

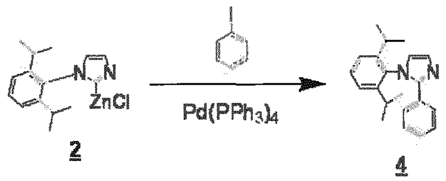


2 4

In the same procedure as in Synthesis Example 2, the compound 2 was prepared from a solution containing the compound 1 (28.5 g, 125 mmol), tetrahydrofuran (125 ml), the n-BuLi-hexane solution (74.9 ml, mole number of n-BuLi in the solution: 125 mmol, molarity (mole number of n-BuLi/solution amount): 1.67M) and zinc chloride (28.4 g, 208 mmol) dissolved in tetrahydrofuran (208 ml). After the preparation of the compound 2, iodobenzene (21.3 g, 104 mmol) and $Pd(PPh_3)_4$ (2.89 g, 2.5 mmol) were put into the three-necked flask containing the compound 2 and refluxed for 16 hours under nitrogen atmosphere. In short, in Comparative 2, the solvent removal step was not implemented and toluene was not added.

A relationship of the solvent amounts in the reaction system in Synthesis Example 12 is shown in Table 1. A relationship between the total volume $V_A$ [liter] of tetrahydrofuran (i.e., the ether solvent having at most 5 carbon atoms) and the mole number $N_{f2}$ [mol] of iodobenzene is represented below according the same calculation as in Synthesis Example 3 as shown in Table 1.

$$V_A/N_{f2}=3.2$$

The relationship of the numerical formula (1) was not satisfied.

After the reaction, a small amount of water was added to the sample to deactivate the reaction. The mixture was diluted with dichloromethane (300 ml). An aqueous solution of tetrasodium ethylenediaminetetraacetate dihydrate (129.9 g, 312 mmol) was added to the diluted mixture and stirred in a separating funnel. An aqueous sodium hydroxide was further added thereto to adjust an aqueous phase to pH10 or more. A dichloromethane phase was collected. The aqueous phase was extracted with dichloromethane several times. The extract was dried with anhydrous magnesium sulfate, filtrated and concentrated. However, the presence of the target product was not confirmed by thin-layer chromatography.

Comparative 3: Synthesis of Imidazole Compound (Compound 5)

Synthesis Example 13

[Formula 31]

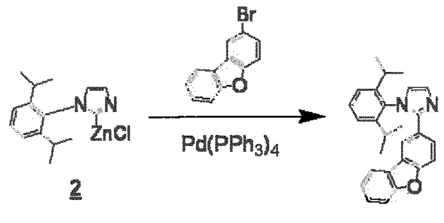


2

5

In the same procedure as in Synthesis Example 2, the compound 2 was prepared from a solution containing the compound 1 (15.98 g, 70 mmol), tetrahydrofuran (140 ml), the n-BuLi-hexane solution (41.9 ml, mole number of n-BuLi in the solution: 70 mmol, molarity (mole number of n-BuLi/solution amount): 1.67M) and zinc chloride (13.6 g, 100 mmol) dissolved in tetrahydrofuran (100 ml). After the preparation of the compound 2, 2-Bromodibenzofuran (12.35 g, 50 mmol) and $Pd(PPh_3)_4$ (2.89 g, 2.5 mmol) were put into the three-necked flask containing the compound 2 and refluxed for 18 hours under nitrogen atmosphere. In short, in Comparative 3, the solvent removal step was not implemented and toluene was not added.

A relationship of the solvent amounts in the reaction system in Synthesis Example 13 is shown in Table 1. A relationship between the total volume $V_A$ [liter] of tetrahydrofuran (i.e., the ether solvent having at most 5 carbon atoms) and the mole number $N_{f2}$ [mol] of 2-Bromodibenzofuran is represented below according the same calculation as in Synthesis Example 3 as shown in Table 1.

$$V_A/N_{f2}=4.8$$

The relationship of the numerical formula (1) was not satisfied.

After the reaction, a small amount of water was added to the sample to deactivate the reaction. The mixture was diluted with dichloromethane (300 ml). An aqueous solution of tetrasodium ethylenediaminetetraacetate dihydrate (62.4 g, 150 mmol) was added to the diluted mixture and stirred in a separating funnel. An aqueous sodium hydroxide was further added thereto to adjust an aqueous phase to pH10 or more. A dichloromethane phase was collected. The aqueous phase was extracted with dichloromethane several times. The extract was dried with anhydrous magnesium sulfate, filtrated and concentrated. However, the presence of the target product was not confirmed by thin-layer chromatography.

Comparative 4: Synthesis of Imidazole Compound (Compound 9)

Synthesis Example 14

[Formula 32]

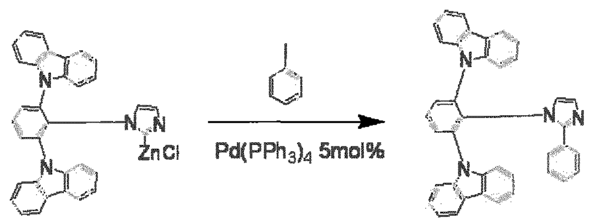


8

9

In the same procedure as in Synthesis Example 9, the compound 8 was prepared from a solution containing the compound 7 (5.69 g, 12 mmol), tetrahydrofuran (72 ml), the n-BuLi-hexane solution (7.2 ml, mole number of n-BuLi in the solution: 12 mmol, molarity (mole number of n-BuLi/solution amount): 1.67M) and zinc chloride (2.04 g, 15 mmol) dissolved in tetrahydrofuran (15 ml). After the preparation of the compound 8, iodobenzene (2.04 g, 10 mmol) and Pd $(PPh_3)_4$ (231 mg, 0.2 mmol) were added to the three-necked flask containing the compound 8 and refluxed for 16 hours

under nitrogen atmosphere. In short, in Comparative 4, the solvent removal step was not implemented and toluene was not added.

A relationship of the solvent amounts in the reaction system in Synthesis Example 14 is shown in Table 1. A relationship between the total volume $V_A$ [liter] of tetrahydrofuran (i.e., the ether solvent having at most 5 carbon atoms) and the mole number $N_{f2}$ [mol] of iodobenzene is represented below according the same calculation as in Synthesis Example 3 as shown in Table 1.

$$V_A/N_{f2}=(72+15)\times 10^{-3}/10\times 10^{-3}=8.7$$

The relationship of the numerical formula (1) was not satisfied.

After the reaction, a small amount of water was added to the sample to deactivate the reaction. The mixture was diluted with dichloromethane (200 ml). An aqueous solution of tetrasodium ethylenediaminetetraacetate dihydrate (8.32 g, 20 mmol) was added to the diluted mixture and stirred in a separating funnel. An aqueous sodium hydroxide was further added thereto to adjust an aqueous phase to pH10 or more. A dichloromethane phase was collected. The aqueous phase was extracted with dichloromethane several times. The extract was dried with anhydrous magnesium sulfate, filtrated and concentrated. In Synthesis Example 14, the compound 9 was not confirmed according to an analysis result of high speed liquid chromatography.

Liquid Chromatography Analysis

Before refinement by silica-gel chromatography, the samples obtained in the synthesis of the compound 9 in Example 4 and Comparative 4 each were used to prepare a 100-ml tetrahydrofuran solution using a 100-ml measuring cylinder. Subsequently, a step to dilute the solution to have one-tenth concentration (specifically, a step in which 1 ml was taken out from the solution and used for preparing a 10-ml tetrahydrofuran solution using a 10-ml measuring cylinder) was repeated three times. Thus, a 10-ml solution having a concentration substantially equivalent to that of a 100-L tetrahydrofuran solution in which the sample was dissolved was prepared.
Figs. 3A and 3B show analysis results of this solution by high speed liquid chromatography.
Analysis Conditions by High Speed Liquid Chromatography
Measurement was made under the following condition using a high speed liquid chromatography device (manufactured by Agilent Technologies: Agilent 1100 series (model: Binary P ump G1312A).
Column: Inertsil ODS3V (ø 4.6 mm×250 mm, 5μm)
Mobile Phase: 0.1 mass% HCOOH + 0.1 mass% $HCOONH_4$ aqueous solution / acetonitrile=30/70 (v/v) (volume ratio)
Flow Rate: 1000 μl/min
Injected amount: 5.0 μl
UV detection wavelength: 254 nm

Mass Analysis

Mass analysis was conducted, so that peak components in the chart were confirmed to be the compound 7 (i.e., raw materials) and the target compound 9.

According to the results of the high speed liquid chromatography analysis and the mass analysis, the compound 9 can be synthesized at a relatively favorable yield rate in the invention whereas the compound 9 cannot be synthesized by a conventional method as shown in Comparatives.

Table 2 shows the yield rates of the compounds 3, 4, 5 and 9 synthesized in Examples 1 to 4 and Comparatives 1 to 4.

Table 2

| | Yield Rate | |
|---|---|---|
| Target Compound | Examples | Comparatives |
| Compound 3 | 84% (Example 1) | 4% (Comparative 1) |
| Compound 4 | 78% (Example 2) | unconfirmed *1 (Comparative 2) |
| Compound 5 | 80% (Example 3) | unconfirmed *1 (Comparative 3) |

(continued)

| | Yield Rate | |
|---|---|---|
| Target Compound | Examples | Comparatives |
| Compound 9 | 42% (Example 4) | unconfirmed *2 (Comparative 4) |
| *1 No target compound was confirmed by thin-layer chromatography<br>*2 No target compound was confirmed by HPLC. | | |

As shown in Table 2, even though the target compound is the same, the target compound is synthesized at an extremely low yield rate or cannot be synthesized according to the synthesis method of Comparatives, whereas the target compound can be synthesized at a high yield rate according to the synthesis method of Examples.
Accordingly, the manufacturing method of the imidazole compound in the invention is recognized as an extremely useful manufacturing method for synthesizing the imidazole compound represented by the formula (1).

Relationship between the Invention and Prior Art

As described above, the invention can provide the imidazole compound incapable of being synthesized by a conventional method.
The imidazole compound incapable of being synthesized by the synthesis methods disclosed in Patent Literatures 1 and 2 is not disclosed as being obviously manufacturable by those skilled in the art with reference to technical knowledge at the time of filing the application. Accordingly, the imidazole compound incapable of being synthesized by the synthesis methods disclosed in Patent Literatures 1 and 2 cannot be "Cited Invention" against the invention.
According to the court case (Heisei 11, (High court, Administrative lawsuit) 285), "[...] when the invention is incomplete or unimplementable for some reasons, it is natural that the invention be not regarded as an already-existing invention to serve as a criterion for judging novelty. Accordingly, it can be said that, in order to be regarded as "the invention disclosed in distributed publications," the invention needs to be implementable by those skilled in the art". In consideration based on the court case, the imidazole compound incapable of being synthesized in Patent Literatures 1 and 2 cannot be "Cited Invention."
The same judgment was made also in U.S.A (In re WIGGINS, CCPA 1973) and in Europe (T206/83 (0J1987,5)).

## Claims

**1.** A manufacturing method of an imidazole compound represented by a formula (1) below, the method comprising:

reacting a compound represented by a formula (2) below with a compound represented by a formula (3) below, wherein
when reacting the compound represented by the formula (2) with the compound represented by the formula (3), in a reaction system, a mole number $N_{f2}$ [mol] of the compound represented by the formula (2) and a total volume $V_A$ [liter] of an ether solvent comprising at most 5 carbon atoms satisfy a relationship of a numerical formula (1) below,

[Formula 1]

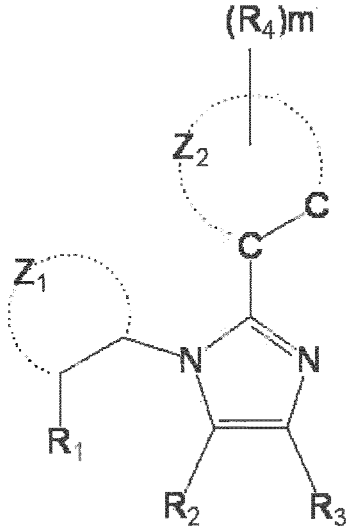

(1)

wherein:

$R_1$ represents a hydrogen atom or a substituent;

$Z_1$ represents a group of atoms necessary for forming a hydrocarbon cyclic group or a heterocyclic group, the hydrocarbon cyclic group or the heterocyclic group formed in $Z_1$ comprising at least one substituent represented by $R_1$;

$R_2$ and $R_3$ represent a bond, a hydrogen atom or an aromatic hydrocarbon group and mutually bonded to form a five-membered hydrocarbon ring, six-membered hydrocarbon ring, five-membered heterocyclic ring or six-membered heterocyclic ring, the rings optionally further comprising a substituent;

$Z_2$ represents a group of atoms necessary for forming a five-membered hydrocarbon ring, six-membered hydrocarbon ring, five-membered heterocyclic ring or six-membered heterocyclic ring together with C-C;

$R_4$ represents a hydrogen atom or a substituent; and

m is an integer of 1 to 5,

[Formula 2]

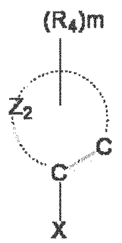

(2)

wherein:

X represents a halogen atom; and

$Z_2$, $R_4$ and m represent the same as those in the formula (1), and

[Formula 3]

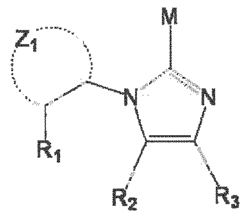

(3)

wherein:

M represents a boron atom, a magnesium atom, a silicon atom, a stannum atom and a zinc atom and optionally further has a substituent; and

$Z_1$, $R_1$, $R_2$ and $R_3$ represent the same as those in the formula (1) [Numerical Formula 1]

$$V_A/N_{f2} \leq 3 \qquad (1).$$

2. The manufacturing method of the imidazole compound according to claim 1, wherein the mole number $N_{f2}$ of the compound represented by the formula (2) and the total volume $V_A$ satisfy a relationship of a numerical formula (2) below,
[Numerical Formula 2]

$$V_A/N_{f2} \leq 2 \qquad (2).$$

3. The manufacturing method of the imidazole compound according to claim 1, wherein the mole number $N_{f2}$ of the compound represented by the formula (2) and the total volume $V_A$ satisfy a relationship of a numerical formula (3) below,
[Numerical Formula 3]

$$V_A/N_{f2} \leq 1 \qquad (3).$$

4. The manufacturing method of the imidazole compound according to any one of claims 1 to 3, wherein the ether solvent comprising at most 5 carbon atoms is at least one ether solvent selected from tetrahydrofuran, tetrahydropyran, 1,4-dioxane, 1,3-dioxane, diethylether and 1,2-dimethoxyethane.

5. The manufacturing method of the imidazole compound according to any one of claims 1 to 4, further comprising:

implementing a solvent removal treatment of removing the solvent in the reaction system to adjust the relationship between the mole number $N_{f2}$ and the total volume $V_A$.

6. The manufacturing method of the imidazole compound according to any one of claims 1 to 5, wherein as a second solvent, at least one solvent selected from an aliphatic hydrocarbon solvent comprising at least 7 carbon atoms, an aromatic hydrocarbon solvent and an ether solvent comprising at least 6 carbon atoms is contained in the reaction system, and
when reacting the compound represented by the formula (2) with the compound represented by the formula (3), in the reaction system, the mole number $N_{f2}$ of the compound represented by the formula (2) and a total volume $V_B$ of the second solvent satisfy a relationship of a numerical formula (4) below,
[Numerical Formula 4]

$$0.1 \leq V_B/N_{f2} \qquad (4).$$

7. The manufacturing method of the imidazole compound according to claim 6, wherein

in the reaction system, the mole number $N_{f2}$ of the compound represented by the formula (2) and the total volume $V_B$ of the second solvent satisfy a relationship of a numerical formula (5) below,
[Numerical Formula 5]

$$0.1 \leq V_B/N_{f2} \leq 10 \qquad (5).$$

**8.** The manufacturing method of the imidazole compound according to claim 6 or 7, wherein
the ether solvent comprising at least 6 carbon atoms is at least one ether solvent selected from dipropylether, dibutylether, diethylene glycol dimethyl ether, diethylene glycol diethyl ether, methoxybenzene, ethoxybenzene, methyl anisole, ethyl anisole, dimethoxybenzene and methoxyethoxybenzene.

**9.** The manufacturing method of the imidazole compound according to any one of claims 6 to 8, wherein
carbon atoms of the aliphatic hydrocarbon solvent comprising at least 7 carbon atoms are in a range of 7 to 50, and carbon atoms of the aromatic hydrocarbon solvent are in a range of 6 to 20.

**10.** The manufacturing method of the imidazole compound according to any one of claims 6 to 9, wherein
the aromatic hydrocarbon solvent is at least one solvent selected from benzene, toluene, xylene, ethylbenzene, trimethylbenzene and tetramethylbenzene.

**11.** The manufacturing method of the imidazole compound according to any one of claims 1 to 10, wherein
in the formula (3), M is a substituted or unsubstituted zinc atom.

**12.** An imidazole compound manufactured by the manufacturing method of the imidazole compound according to any one of claims 1 to 11.

**13.** An imidazole compound comprising a substituent represented by $R_1$ and comprising a steric parameter (Es) value of -2.0 or less in a formula (1) below,

[Formula 4]

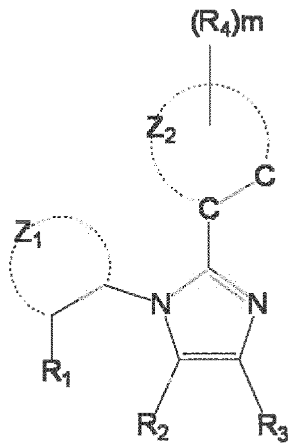

(1)

wherein:

$Z_1$ represents a group of atoms necessary for forming a hydrocarbon cyclic group or a heterocyclic group, the hydrocarbon cyclic group or the heterocyclic group formed in $Z_1$ comprising at least one substituent represented by $R_1$;
$R_2$ and $R_3$ represent a bond, a hydrogen atom or an aromatic hydrocarbon group and mutually bonded to form a five-membered hydrocarbon ring, six-membered hydrocarbon ring, five-membered heterocyclic ring or six-membered heterocyclic ring, the rings optionally further comprising a substituent;

$Z_2$ represents a group of atoms necessary for forming a five-membered hydrocarbon ring, six-membered hydrocarbon ring, five-membered heterocyclic ring or six-membered heterocyclic ring together with C-C;
$R_4$ represents a hydrogen atom or a substituent; and
m is an integer of 1 to 5.

**14.** The imidazole compound according to claim 13, wherein
$R_1$ represents a substituent comprising a steric parameter (Es) value of -2.5 or less.

**15.** The imidazole compound according to claim 13, wherein
$R_1$ represents a substituent comprising a steric parameter (Es) value of -3.0 or less.

**16.** The imidazole compound according to claim 13, wherein
$R_1$ represents a substituent comprising a steric parameter (Es) value of -5.0 or less.

**17.** The imidazole compound according to any one of claims 13 to 16, wherein
$R_1$ represents a substituent comprising a molecular weight of 43 or more.

**18.** The imidazole compound according to any one of claims 13 to 16, wherein
$R_1$ represents a substituent comprising a molecular weight of 77 or more.

**19.** The imidazole compound according to any one of claims 13 to 16, wherein
$R_1$ represents a substituent comprising a molecular weight of 116 or more.

**20.** The imidazole compound according to any one of claims 13 to 16, wherein
$R_1$ represents a substituent comprising a molecular weight of 166 or more.

**21.** An imidazole-based compound comprising the imidazole compound according to any one of claims 13 to 20 as a partial structure.

**22.** An organic metal complex comprising the imidazole compound according to any one of claims 13 to 20 as a partial structure.

**23.** The organic metal complex according to claim 22, further comprising:

at least one metal selected from metal elements from the eighth group to the eleventh group in the periodic table.

**24.** An organic-electroluminescence-device material comprising:

at least one of the imidazole compound according to any one of claims 13 to 20, the imidazole-based compound according to claim 21 and the organic metal complex according to claim 22 or 23.

**25.** An organic electroluminescence device comprising:

a cathode;
an anode; and
a plurality of organic compound layers provided between the cathode and the anode, the organic compound layers comprising an emitting layer, wherein
at least one layer of the organic compound layers comprises the organic-electroluminescence-device material according to claim 24.

**26.** A display device comprising the organic electroluminescence device according to claim 25.

**27.** A lighting device comprising the organic electroluminescence device according to claim 25.

FIG. 1

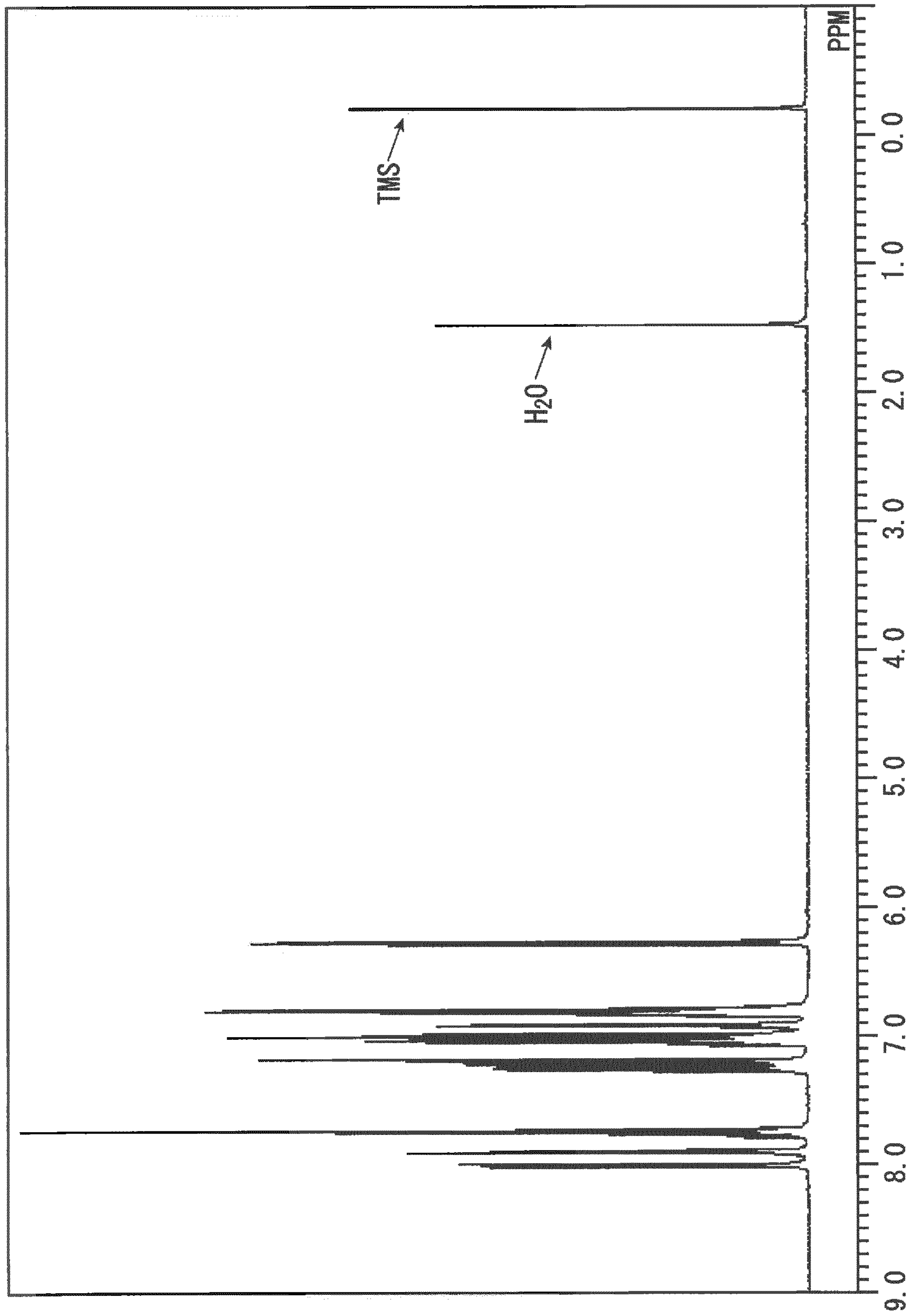

FIG.2

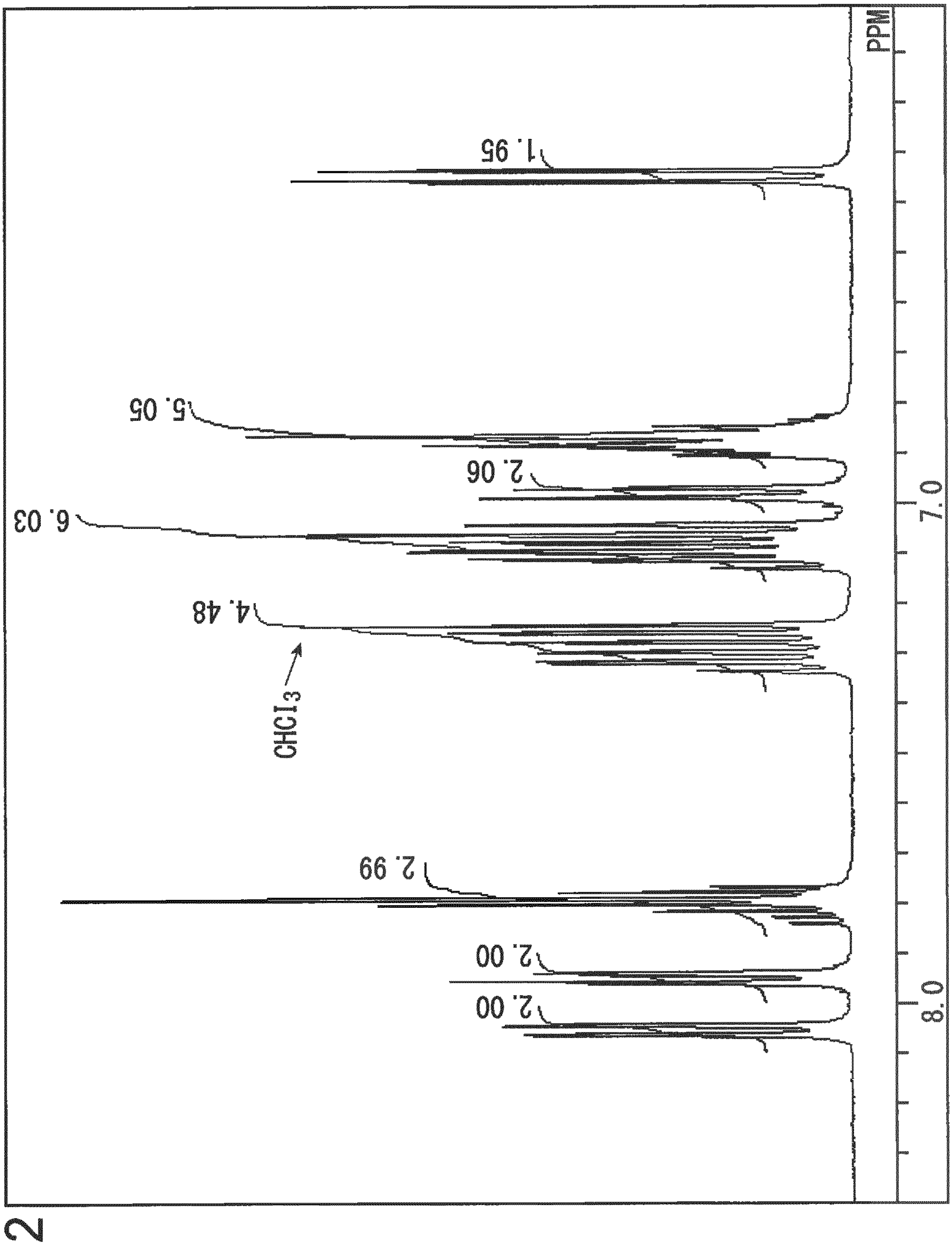

FIG.3A

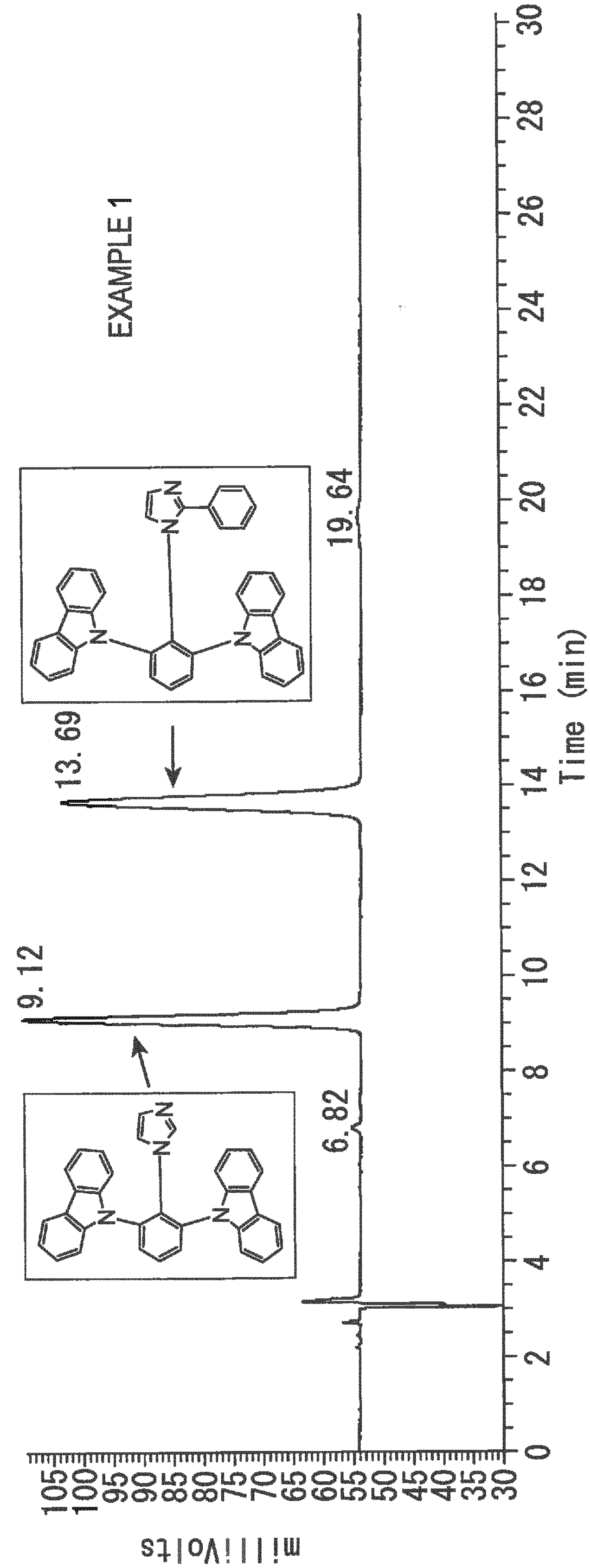
EXAMPLE 1
19.64
13.69
9.12
6.82
Time (min)
milliVolts
0
2
4
6
8
10
12
14
16
18
20
22
24
26
28
30
105
100
95
90
85
80
75
70
65
60
55
50
45
40
35
30

FIG. 3B

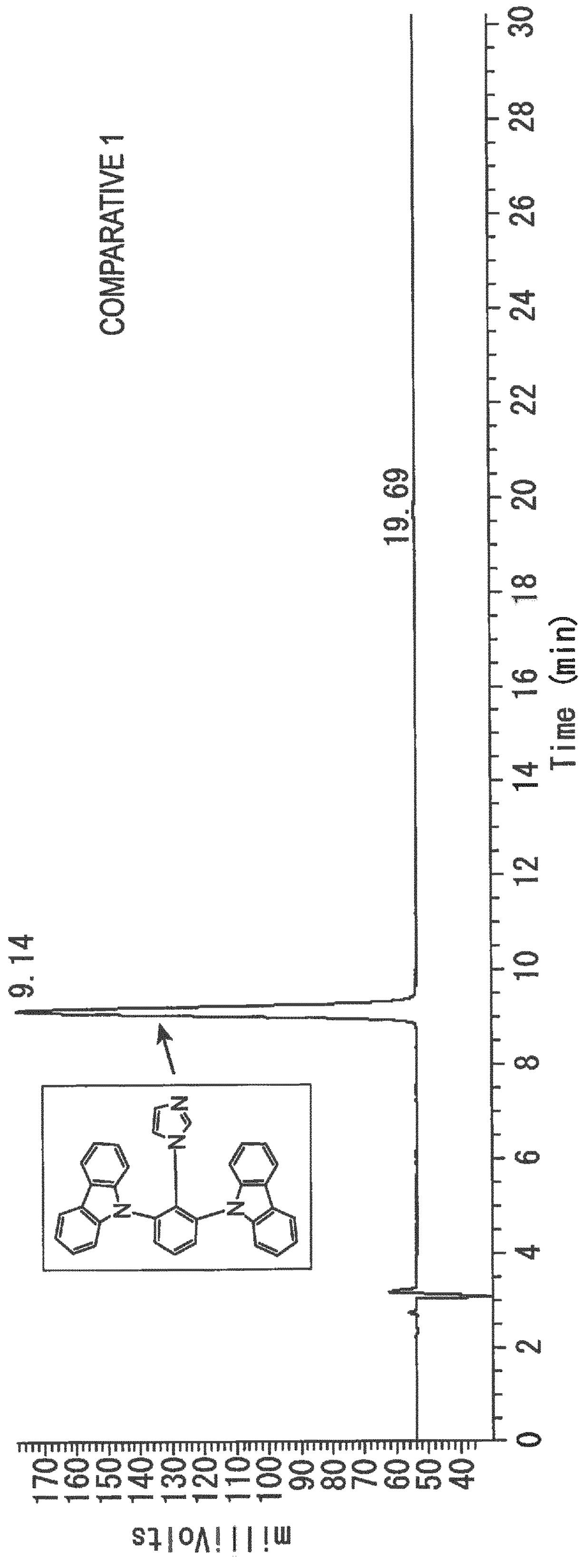
COMPARATIVE 1
9.14
19.69
milliVolts
170
160
150
140
130
120
110
100
90
80
70
60
50
40
0
2
4
6
8
10
12
14
16
18
20
22
24
26
28
30
Time (min)

**INTERNATIONAL SEARCH REPORT**

International application No. PCT/JP2012/050087

A. CLASSIFICATION OF SUBJECT MATTER
*C07D233/58*(2006.01)i, *C07D403/04*(2006.01)i, *C07D403/14*(2006.01)i, *C07D405/04*(2006.01)i, *C09K11/06*(2006.01)i, *H01L51/50*(2006.01)i

According to International Patent Classification (IPC) or to both national classification and IPC

B. FIELDS SEARCHED

Minimum documentation searched (classification system followed by classification symbols)
C07D233/58, C07D403/04, C07D403/14, C07D405/04, C09K11/06, H01L51/50

Documentation searched other than minimum documentation to the extent that such documents are included in the fields searched

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X/A | WO 2006/121811 A1 (Universal Display Corp.), 16 November 2006 (16.11.2006), examples & JP 2008-542203 A & US 2006/0251923 A1 & US 2007/0088167 A1 & EP 1878053 A1 & CN 101180304 A & KR 10-2008-0007570 A | 12-19,21-27/ 1-11,20 |
| X/A | JP 2005-68110 A (Mitsubishi Chemical Corp.), 17 March 2005 (17.03.2005), paragraph [0232], Object 4 (Family: none) | 12/1-11,20 |
| X/A | JP 2008-303150 A (Konica Minolta Holdings, Inc.), 18 December 2008 (18.12.2008), examples (Family: none) | 12/1-11,20 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 February, 2012 (09.02.12) | 21 February, 2012 (21.02.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/050087

C (Continuation). DOCUMENTS CONSIDERED TO BE RELEVANT

| Category* | Citation of document, with indication, where appropriate, of the relevant passages | Relevant to claim No. |
|---|---|---|
| X/A | BELLINA Fabio et al., Regioselective synthesis of 1,5-diaryl-1H-imidazoles by palladium-catalyzed direct arylation of 1-aryl-1H-imidazoles, Journal of Organic Chemistry, 2005, 70(10), pp.3997-4005, compound 7a | 12/1-11,20 |
| X/A | WO 2004/009604 A2 (Therasense), 29 January 2004 (29.01.2004), example 2<br>& JP 2006-509837 A & US 2003/0096997 A1<br>& US 2004/0099529 A1 & US 2006/0149067 A1<br>& US 2008/0197026 A1 & US 2009/0095642 A1<br>& US 2009/0143584 A1 & US 2010/0038242 A1<br>& EP 1507785 A & EP 2301942 A1<br>& CN 1620462 A | 12/1-11,20 |
| X/A | JP 3-192366 A (Mitsubishi Paper Mills Ltd.), 22 August 1991 (22.08.1991), page 15, upper left column (Family: none) | 12/1-11,20 |
| P,X | US 2011/0057559 A1 (Universal Display Corp.), 10 March 2011 (10.03.2011), (Family: none) | 12-14,17, 21-27 |
| P,A | KR 10-2011-0079401 A (CS Elsolar Co. Ltd.), 07 July 2011 (07.07.2011), (Family: none) | 1-11 |

Form PCT/ISA/210 (continuation of second sheet) (July 2009)

**INTERNATIONAL SEARCH REPORT**

International application No.
PCT/JP2012/050087

<Subject of search>

The inventions described in claims 1-27 of the present application relate to an imidazole compound represented by general formula (1) of the present application, and the imidazole compound represented by general formula (1) of the present invention includes great many compounds within the scope thereof.

However, those compounds which are supported by the description in the meaning within PCT Article 6 and disclosed in the meaning within PCT Article 5 are just some of the claimed compounds.

Such being the case, the search was carried out on the parts which are supported by and disclosed in the description, i.e., compounds represented by general formula (1) of the present application wherein each of $R_2$ and $R_3$ represents a hydrogen atom and the ring containing $Z_1$ is a phenyl.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008542203 A
- JP 2008303150 A


### Non-patent literature cited in the description


- Structure-activity relationships of drug: The significance in drug design and mode-of-action studies. Nankodo Co., Ltd, 1979, 124-126
- American Chemical Society Professional Reference Book. Exploring QSAR. 81
- **UNGER, S.H. ; HANSCH, C.** *Prog. Phys. Org. Chem.,* 1976, vol. 12, 91
- Surfactant-Property, Application, Chemical Ecology. Kodansha Ltd, 1979
- New Surfactant. SANKYO PUBLISHING Co., Ltd, 1986